# EUROPEAN PATENT SPECIFICATION

(11) **EP 1 541 689 B1**
(45) Date of publication and mention of the grant of the patent: **05.09.2012**
(21) Application number: 05004142.5
(22) Date of filing: 20.02.1997
(51) Int. Cl.: C12N 15/62, C12N 15/16, C07K 14/59, C07K 14/715, C07K 14/72, C07K 16/46, C12N 15/85, C12N 5/10, A61K 38/24, C12N 15/13, A61K 39/395

(54) **Hybrid proteins which form heterodimers**
Heterodimerebildende Hybrid-Proteine
Protéines hybrides qui forment des hétérodimères

(30) Priority: 20.02.1996 US 11936 P
(43) Date of publication of application: 15.06.2005
(62) Divisional of application: 97906604.0
(73) Proprietor: Merck Serono SA, 1267 Coinsins, Vaud (CH)
(72) Inventor: CAMPBELL, Robert K., Wrentham, MA 02093 (US); JAMESON, Bradford A., Milton, MA 02186 (US); CHAPPEL, Scott C., Milton, MA 02186 (US)
(74) Representative: Vossius & Partner

(56) References cited:
- WO-A2-2007/062037
- GREGORY A. JOHNSON ET AL.: "Baculovirus-Insect cell production of bioactive Choriogonadotropin-Immunoglobulin G heavy-chain fusion proteins in sheep" BIOLOGY OF REPRODUCTION, vol. 52, no. 1, January 1995 (1995-01), pages 68-73, XP000675391
- NARAYAN, PREMA ET AL: "Functional expression of yoked human chorionic gonadotropin in baculovirus-infected insect cells" MOL. ENDOCRINOL. (1995), 9(12), 1720-6 CODEN: MOENEN;ISSN: 0888-8809, 1995, XP000675344
- FRANCIS J. MORGAN ET AL.: "The amino acid sequence of human chorionic gonadotropin. The alpha subunit and beta subunit", THE JOURNAL OF BIOLOGICAL CHEMISTRY, vol. 250, no. 13, 1975, pages 5247-5254,

### SEQUENCE LISTING

(1) GENERAL INFORMATION:
   (i) APPLICANT:
      (A) NAME: Applied Research Systems ARS Holding N.V.
      (B) STREET: 14 John B. Gorsiraweg
      (C) CITY: Curacao
      (E) COUNTRY: Netherlands Antilles
      (F) POSTAL CODE (ZIP):
         (A) NAME: CAMPBELL, Robert C.
         (B) STREET: 25 Meadowbrook Drive
         (C) CITY: Wrentham
         (E) STATE: Massachusetts
         (F) COUNTRY: United States of America
         (A) NAME: JAMESON, Bradford A.
         (B) STREET: 76 Robbins Street
         (C) CITY: Milton
         (E) STATE: Massachusetts
         (F) COUNTRY: United States of America
         (A) NAME: CHAPPEL, Scott C.
         (B) STREET: 125 Canton Avenue
         (C) CITY: Milton
         (E) STATE: Massachusetts
         (F) COUNTRY: United States of America
   (ii) TITLE OF INVENTION: HYBRID PROTEINS
   (iii) NUMBER OF SEQUENCES: 22
   (iv) CORRESPONDENCE ADDRESS:
      (A) ADDRESSEE: BROWDY AND NEIMARK
      (B) STREET: 419 Seventh Street N.W., Ste. 300
      (C) CITY: Washington
      (D) STATE: D.C.
      (E) COUNTRY: USA
      (F) ZIP: 22207
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) PRIOR APPLICATION DATA:
      (A) APPLICATION NUMBER: 60/011,936
      (B) FILING DATE: 20 February 1996
      (C) CLASSIFICATION:
   (viii) ATTORNEY/AGENT INFORMATION:
      (A) NAME: Browdy, Roger L.
      (B) REGISTRATION NUMBER: 25,618
      (C) REFERENCE/DOCKET NUMBER: CAMPBELL=2A PCT
   (ix) TELECOMMUNICATION INFORMATION:
      (A) TELEPHONE: (202) 628-5197
      (B) TELEFAX: (202) 737-3528
(2) INFORMATION FOR SEQ ID NO:1:
   (i) SEQUENCE CHARACTERISTICS:
      (A) LENGTH: 1049 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 278..1047
   (xi) SEQUENCE DESCRIPTION: SEQ ID NO:1:
   TCCACATGGC TACAGGTAAG CGCCCCTAAA ATCCCTTTGG GCACAATGTG TCCTGAGGGG     60
   AGAGGCAGCG ACCTGTAGAT GGGACGGGGG CACTAACCCT CAGGTTTGGG GCTTCTCAAT    120
   CTCACTATCG CCATGTAAGC CCAGTATTTG GCCAATCTCA GAAAGCTCCT CCTCCCTGGA    180
   GGGATGGAGA GAGAAAAACA AACAGCTCCT GGAGCAGGGA GAGTGCTGGC CTCTTGCTCT    240
   CCGGCTCCCT CTGTTGCCCT CTGGTTTCTC CCCAGGC TCC CGG ACG TCC CTG CTC     295
                                            Ser Arg Thr Ser Leu Leu
                                             1               5
   CTG GCT TTT GGC CTG CTC TGC CTG CCC TGG CTT CAA GAG GGC AGT GCC      343
   Leu Ala Phe Gly Leu Leu Cys Leu Pro Trp Leu Gln Glu Gly Ser Ala
               10                  15                  20
   GAT AGT GTG TGT CCC CAA GGA AAA TAT ATC CAC CCT CAA AAT AAT TCC      391
   Asp Ser Val Cys Pro Gln Gly Lys Tyr Ile His Pro Gln Asn Asn Ser
           25                  30                  35
   ATT TGC TGT ACC AAG TGC CAC AAA GGA ACC TAC TTG TAC AAT GAC TGT      439
   Ile Cys Cys Thr Lys Cys His Lys Gly Thr Tyr Leu Tyr Asn Asp Cys
       40                  45                  50
   CCA GGC CCG GGG CAG GAT ACG GAC TGC AGG GAG TGT GAG AGC GGC TCC      487
   Pro Gly Pro Gly Gln Asp Thr Asp Cys Arg Glu Cys Glu Ser Gly Ser
   55                  60                  65                  70
   TTC ACC GCT TCA GAA AAC CAC CTC AGA CAC TGC CTC AGC TGC TCC AAA      535
   Phe Thr Ala Ser Glu Asn His Leu Arg His Cys Leu Ser Cys Ser Lys
                   75                  80                  85
   TGC CGA AAG GAA ATG GGT CAG GTG GAG ATC TCT TCT TGC ACA GTG GAC      583
   Cys Arg Lys Glu Met Gly Gln Val Glu Ile Ser Ser Cys Thr Val Asp
               90                  95                  100
   CGG GAC ACC GTG TGT GGC TGC AGG AAG AAC CAG TAC CGG CAT TAT TGG      631
   Arg Asp Thr Val Cys Gly Cys Arg Lys Asn Gln Tyr Arg His Tyr Trp
           105                 110                 115
   AGT GAA AAC CTT TTC CAG TGC TTC AAT TGC AGC CTC TGC CTC AAT GGG      679
   Ser Glu Asn Leu Phe Gln Cys Phe Asn Cys Ser Leu Cys Leu Asn Gly
       120                 125                 130
   ACC GTG CAC CTC TCC TGC CAG GAG AAA CAG AAC ACC GTG TGC ACC TGC      727
   Thr Val His Leu Ser Cys Gln Glu Lys Gln Asn Thr Val Cys Thr Cys
   135                 140                 145                 150
   CAT GCA GGT TTC TTT CTA AGA GAA AAC GAG TGT GTC TCC TGT GCC GGT      775
   His Ala Gly Phe Phe Leu Arg Glu Asn Glu Cys Val Ser Cys Ala Gly
                   155                 160                 165
   GCT GCC CCA GGT TGC CCA GAA TGC ACG CTA CAG GAA AAC CCA TTC TTC      823
   Ala Ala Pro Gly Cys Pro Glu Cys Thr Leu Gln Glu Asn Pro Phe Phe
               170                 175                 180
   TCC CAG CCG GGT GCC CCA ATA CTT CAG TGC ATG GGC TGC TGC TTC TCT        871
   Ser Gln Pro Gly Ala Pro Ile Leu Gln Cys Met Gly Cys Cys Phe Ser
           185                 190                 195
   AGA GCA TAT CCC ACT CCA CTA AGG TCC AAG AAG ACG ATG TTG GTC CAA        919
   Arg Ala Tyr Pro Thr Pro Leu Arg Ser Lys Lys Thr Met Leu Val Gln
       200                 205                 210
   AAG AAC GTC ACC TCA GAG TCC ACT TGC TGT GTA GCT AAA TCA TAT AAC        967
   Lys Asn Val Thr Ser Glu Ser Thr Cys Cys Val Ala Lys Ser Tyr Asn
   215                 220                 225                 230
   AGG GTC ACA GTC ATG GGG GGT TTC AAA GTG GAG AAC CAC ACG GGG TGC       1015
   Arg Val Thr Val Met Gly Gly Phe Lys Val Glu Asn His Thr Gly Cys
                   235                 240                 245
   CAC TGC AGT ACT TGT TAT TAT CAC AAA TCT  TA AG                        1049
   His Cys Ser Thr Cys Tyr Tyr His Lys Ser
               250                 255
(2) INFORMATION FOR SEQ ID NO:2:
   (i) SEQUENCE CHARACTERISTICS:
      (A) LENGTH: 256 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Ser Arg Thr Ser Leu Leu Leu Ala Phe Gly Leu Leu Cys Leu Pro Trp
    1               5                  10                  15
   Leu Gln Glu Gly Ser Ala Asp Ser Val Cys Pro Gln Gly Lys Tyr Ile
               20                  25                  30
   His Pro Gln Asn Asn Ser Ile Cys Cys Thr Lys Cys His Lys Gly Thr
           35                  40                  45
   Tyr Leu Tyr Asn Asp Cys Pro Gly Pro Gly Gln Asp Thr Asp Cys Arg
       50                  55                  60
   Glu Cys Glu Ser Gly Ser Phe Thr Ala Ser Glu Asn His Leu Arg His
   65                  70                  75                  80
   Cys Leu Ser Cys Ser Lys Cys Arg Lys Glu Met Gly Gln Val Glu Ile
                   85                  90                  95
   Ser Ser Cys Thr Val Asp Arg Asp Thr Val Cys Gly Cys Arg Lys Asn
               100                 105                 110
   Gln Tyr Arg His Tyr Trp Ser Glu Asn Leu Phe Gln Cys Phe Asn Cys
           115                 120                 125
   Ser Leu Cys Leu Asn Gly Thr Val His Leu Ser Cys Gln Glu Lys Gln
       130                 135                 140
   Asn Thr Val Cys Thr Cys His Ala Gly Phe Phe Leu Arg Glu Asn Glu
   145                 150                 155                 160
   Cys Val Ser Cys Ala Gly Ala Ala Pro Gly Cys Pro Glu Cys Thr Leu
                   165                 170                 175
   Gln Glu Asn Pro Phe Phe Ser Gln Pro Gly Ala Pro Ile Leu Gln Cys
               180                 185                 190
   Met Gly Cys Cys Phe Ser Arg Ala Tyr Pro Thr Pro Leu Arg Ser Lys
           195                 200                 205
   Lys Thr Met Leu Val Gln Lys Asn Val Thr Ser Glu Ser Thr Cys Cys
       210                 215                 220
   Val Ala Lys Ser Tyr Asn Arg Val Thr Val Met Gly Gly Phe Lys Val
   225                 230                 235                     240
   Glu Asn His Thr Gly Cys His Cys Ser Thr Cys Tyr Tyr His Lys Ser
                   245                 250                 255
(2) INFORMATION FOR SEQ ID NO:3:
   (i) SEQUENCE CHARACTERISTICS:
      (A) LENGTH: 1202 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 279..1199
   (xi) SEQUENCE DESCRIPTION: SEQ ID NO:3:
   CTCGAGATGG CTACAGGTAA GCGCCCCTAA AATCCCTTTG GGCACAATGT GTCCTGAGGG      60
   GAGAGGTAGC GACCTGTAGA TGGGACGGGG GCACTAACCC TGAGGTTTGG GGCTTCTGAA     120
   TGTGAGTATC GCCATGTAAG CCCAGTATTT GGCCAATGTC AGAAAGCTCC TGGTCCCTGG     180
   AGGGATGGAG AGAGAAAAAC AAACAGCTCC TGGAGCAGGG AGAGTGCTGG CCTCTTGCTC     240
   TCCGGCTCCC TCTGTTGCCC TGTGGTTTCT CCCCAGGC TCC CGG ACG TCC CTG         293
                                             Ser Arg Thr Ser Leu
                                                             260
   CTC CTG GCT TTT GGC CTG CTC TGC CTG CCC TGG CTT CAA GAG GGC AGT       341
   Leu Leu Ala Phe Gly Leu Leu Cys Leu Pro Trp Leu Gln Glu Gly Ser
               265                 270                 275
   GCC GAT AGT GTG TGT CCC CAA GGA AAA TAT ATC CAC CCT CAA AAT AAT       389
   Ala Asp Ser Val Cys Pro Gln Gly Lys Tyr Ile His Pro Gln Asn Asn
           280                 285                 290
   TCG ATT TGC TGT ACC AAG TGC CAC AAA GGA ACC TAC TTG TAC AAT GAC       437
   Ser Ile Cys Cys Thr Lys Cys His Lys Gly Thr Tyr Leu Tyr Asn Asp
       295                 300                 305
   TGT CCA GGC CCG GGG CAG GAT ACG GAC TGC AGG GAG TGT GAG AGC GGC       485
   Cys Pro Gly Pro Gly Gln Asp Thr Asp Cys Arg Glu Cys Glu Ser Gly
   310                 315                 320                 325
   TCT TTC ACC GCT TCA GAA AAC CAC CTC AGA CAC TGC CTC AGC TGC TCC       533
   Ser Phe Thr Ala Ser Glu Asn His Leu Arg His Cys Leu Ser Cys Ser
                   330                 335                 340
   AAA TGC CGA AAG GAA ATG GGT CAG GTG GAG ATC TCT TCT TGC ACA GTG       581
   Lys Cys Arg Lys Glu Met Gly Gln Val Glu Ile Ser Ser Cys Thr Val
               345                 350                 355
   GAC CGG GAC ACC GTG TGT GGC TGC AGG AAG AAC CAG TAC CGG CAT TAT        629
   Asp Arg Asp Thr Val Cys Gly Cys Arg Lys Asn Gln Tyr Arg His Tyr
           360                 365                 370
   TGG AGT GAA AAC CTT TTC CAG TGC TTC AAT TGC AGC CTC TGC CTC AAT        677
   Trp Ser Glu Asn Leu Phe Gln Cys Phe Asn Cys Ser Leu Cys Leu Asn
       375                 380                 385
   GGG ACC GTG CAC CTC TCC TGC CAG GAG AAA CAG AAC ACC GTG TGC ACC        725
   Gly Thr Val His Leu Ser Cys Gln Glu Lys Gln Asn Thr Val Cys Thr
   390                 395                 400                 405
   TGC CAT GCA GGT TTC TTT CTA AGA GAA AAC GAG TGT GTC TCC TGT GCT        773
   Cys His Ala Gly Phe Phe Leu Arg Glu Asn Glu Cys Val Ser Cys Ala
                   410                 415                 420
   GGT GCT GGT CCA CGG TGC CGC CCC ATC AAT GCC ACC CTG GCT GTG GAG        821
   Gly Ala Gly Pro Arg Cys Arg Pro Ile Asn Ala Thr Leu Ala Val Glu
               425                 430                 435
   AAG GAG GGC TGC CCC GTG TGC ATC ACC GTC AAC ACC ACC ATC TGT GCC        869
   Lys Glu Gly Cys Pro Val Cys Ile Thr Val Asn Thr Thr Ile Cys Ala
           440                 445                 450
   GGC TAC TGC CCC ACC ATG ACC CGC GTG CTG CAG GGG GTC CTC CCC GCC        917
   Gly Tyr Cys Pro Thr Met Thr Arg Val Leu Gln Gly Val Leu Pro Ala
       455                 460                 465
   CTG CCT CAG GTG GTG TGC AAC TAC CGC GAT GTG CGC TTC GAG TCC ATC        965
   Leu Pro Gln Val Val Cys Asn Tyr Arg Asp Val Arg Phe Glu Ser Ile
   470                 475                 480                 485
   CGG CTC CCT GGC TGC CCG CGC GGC GTG AAC CCC GTG GTC TCC TAC GCT       1013
   Arg Leu Pro Gly Cys Pro Arg Gly Val Asn Pro Val Val Ser Tyr Ala
                   490                 495                 500
   GTG GCT CTC AGC TGT CAA TGT GCA CTC TGC CGC CGC AGC ACC ACT GAC       1061
   Val Ala Leu Ser Cys Gln Cys Ala Leu Cys Arg Arg Ser Thr Thr Asp
               505                 510                 515
   TGC GGG GGT CCC AAG GAC CAC CCC TTG ACC TGT GAT GAC CCC CGC TTC       1109
   Cys Gly Gly Pro Lys Asp His Pro Leu Thr Cys Asp Asp Pro Arg Phe
           520                 525                 530
   CAG GAC TCC TCT TCC TCA AAG GCC CCT CCC CCC AGC CTT CCA AGC CCA       1157
   Gln Asp Ser Ser Ser Ser Lys Ala Pro Pro Pro Ser Leu Pro Ser Pro
       535                 540                 545
   TCC CGA CTC CCG GGG CCC TCG GAC ACC CCG ATC CTC CCA CAA TAA           1202
   Ser Arg Leu Pro Gly Pro Ser Asp Thr Pro Ile Leu Pro Gln
   550                 555                 560
(2) INFORMATION FOR SEQ ID NO:4:
   (i) SEQUENCE CHARACTERISTICS:
      (A) LENGTH: 307 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Ser Arg Thr Ser Leu Leu Leu Ala Phe Gly Leu Leu Cys Leu Pro Trp
    1               5                  10                  15
   Leu Gln Glu Gly Ser Ala Asp Ser Val Cys Pro Gln Gly Lys Tyr Ile
               20                  25                  30
   His Pro Gln Asn Asn Ser Ile Cys Cys Thr Lys Cys His Lys Gly Thr
           35                  40                  45
   Tyr Leu Tyr Asn Asp Cys Pro Gly Pro Gly Gln Asp Thr Asp Cys Arg
       50                  55                  60
   Glu Cys Glu Ser Gly Ser Phe Thr Ala Ser Glu Asn His Leu Arg His
   65                  70                  75                  80
   Cys Leu Ser Cys Ser Lys Cys Arg Lys Glu Met Gly Gln Val Glu Ile
                   85                  90                  95
   Ser Ser Cys Thr Val Asp Arg Asp Thr Val Cys Gly Cys Arg Lys Asn
               100                 105                 110
   Gln Tyr Arg His Tyr Trp Ser Glu Asn Leu Phe Gln Cys Phe Asn Cys
           115                 120                 125
   Ser Leu Cys Leu Asn Gly Thr Val His Leu Ser Cys Gln Glu Lys Gln
       130                 135                 140
   Asn Thr Val Cys Thr Cys His Ala Gly Phe Phe Leu Arg Glu Asn Glu
   145                 150                 155                 160
   Cys Val Ser Cys Ala Gly Ala Gly Pro Arg Cys Arg Pro Ile Asn Ala
                   165                 170                 175
   Thr Leu Ala Val Glu Lys Glu Gly Cys Pro Val Cys Ile Thr Val Asn
               180                 185                 190
   Thr Thr Ile Cys Ala Gly Tyr Cys Pro Thr Met Thr Arg Val Leu Gln
           195                 200                 205
   Gly Val Leu Pro Ala Leu Pro Gln Val Val Cys Asn Tyr Arg Asp Val
       210                 215                 220
   Arg Phe Glu Ser Ile Arg Leu Pro Gly Cys Pro Arg Gly Val Asn Pro
   225                 230                 235                 240
   Val Val Ser Tyr Ala Val Ala Leu Ser Cys Gln Cys Ala Leu Cys Arg
                   245                 250                 255
   Arg Ser Thr Thr Asp Cys Gly Gly Pro Lys Asp His Pro Leu Thr Cys
               260                 265                 270
   Asp Asp Pro Arg Phe Gln Asp Ser Ser Ser Ser Lys Ala Pro Pro Pro
           275                 280                 285
   Ser Leu Pro Ser Pro Ser Arg Leu Pro Gly Pro Ser Asp Thr Pro Ile
       290                 295                 300
   Leu Pro Gln
   305
(2) INFORMATION FOR SEQ ID NO:5:
   (i) SEQUENCE CHARACTERISTICS:
      (A) LENGTH: 1147 base pairs
      (B) TYPE: nucleic acid
      (C) STRANDEDNESS: single
      (D) TOPOLOGY: linear
   (ii) MOLECULE TYPE: cDNA
   (ix) FEATURE :
      (A) NAME/KEY: CDS
      (B) LOCATION: 278..1132-;
   (xi) SEQUENCE DESCRIPTION: SEQ ID NO:5:
   TCGAGATGGC TACAGGTAAG CGCCCCTAAA ATCCCTTTGG GCACAATGTG TCCTGAGGGG      60
   AGAGGCAGCG ACCTGTAGAT GGGACGGGGG CACTAACCCT CAGGTTTGGG GCTTTTGAAT     120
   GTGAGTATGG CCATGTAAGC CCAGTATTTG CCCAATCTCA GAAAGCTCCT GGTCCCTGGA     180
   GGGATGGAGA GAGAAAAACA AACAGCTCCT GGAGCAGGGA CACTCCTGGC CTCTTGCTCT     240
   GCGGCTCCGT GTGTTGCCCT GTGGTTTCTC CCCACGC TCC CGG ACG TCC CTG CTC      295
                                           Ser Arg Thr Ser Leu Leu
                                                       310
   CTG GCT TTT GGC CTG CTC TGC CTG CCC TGG CTT CAA GAG GGC AGT GCC      343
   Leu Ala Phe Gly Leu Leu Cys Leu Pro Trp Leu Gln Glu Gly Ser Ala
       315                 320                 325
   GAT AGT GTG TGT CCC CAA GGA AAA TAT ATC CAC CCT CAA AAT AAT TCG      391
   Asp Ser Val Cys Pro Gln Gly Lys Tyr Ile His Pro Gln Asn Asn Ser
   330                 335                 340                 345
   ATT TGC TGT ACC AAG TGC CAC AAA GGA ACC TAC TTG TAC AAT GAC TGT      439
   Ile Cys Cys Thr Lys Cys His Lys Gly Thr Tyr Leu Tyr Asn Asp Cys
                   350                 355                 360
   CCA GGC CCG GGG CAG GAT ACC GAC TGC AGG GAG TGT GAG AGC GGC TCC      487
   Pro Gly Pro Gly Gln Asp Thr Asp Cys Arg Glu Cys Glu Ser Gly Ser
               365                 370                 375
   TTC ACC GCT TCA GAA AAC CAC CTC AGA CAC TGC CTC AGC TGC TCC AAA      535
   Phe Thr Ala Ser Glu Asn His Leu Arg His Cys Leu Ser Cys Ser Lys
           380                 385                 390
   TGC CGA AAG GAA ATG GGT CAG GTG GAG ATC TCT TCT TGC ACA GTG GAC      583
   Cys Arg Lys Glu Met Gly Gln Val Glu Ile Ser Ser Cys Thr Val Asp
       395                 400                 405
   CGG GAC ACC GTG TGT GGC TGC AGG AAG AAC CAG TAC CGG CAT TAT TGG      631
   Arg Asp Thr Val Cys Gly Cys Arg Lys Asn Gln Tyr Arg His Tyr Trp
   410                 415                 420                 425
   AGT GAA AAC CTT TTC CAG TGC TTC AAT TGC ACC CTC TGC CTC AAT GGG      679
   Ser Glu Asn Leu Phe Gln Cys Phe Asn Cys Thr Leu Cys Leu Asn Gly
                   430                 435                 440
   ACC GTG CAC CTC TCC TGT CAG GAG AAA CAG AAC ACC GTC TGC ACC TGC      727
   Thr Val His Leu Ser Cys Gln Glu Lys Gln Asn Thr Val Cys Thr Cys
               445                 450                 455
   CAT GCA GGT TTC TTT CTA AGA GAA AAC GAG TGT GTC TCC TGT AGT AAC      775
   His Ala Gly Phe Phe Leu Arg Glu Asn Glu Cys Val Ser Cys Ser Asn
           460                 465                 470
   TGT AAG AAA AGC CTG GAG TGC ACG AAG TTG TCC CTA CCC CAG ATT GAG      823
   Cys Lys Lys Ser Leu Glu Cys Thr Lys Leu Ser Leu Pro Gln Ile Glu
       475                 480                 485
   AAT GTT AAG GGC ACT GAG GAC TCA GGC ACC ACA GCC GGT GCT GCC CCA      871
   Asn Val Lys Gly Thr Glu Asp Ser Gly Thr Thr Ala Gly Ala Ala Pro
   490                 495                 500                 505
   GGT TGC CCA GAA TGC ACG CTA CAG GAA AAC CCA TTC TTC TCC CAG CCG        919
   Gly Cys Pro Glu Cys Thr Leu Gln Glu Asn Pro Phe Phe Ser Gln Pro
                   510                 515                 520
   GGT GCC CCA ATA CTT CAG TGC ATG GGC TGC TGC TTC TCT AGA GCA TAT        967
   Gly Ala Pro Ile Leu Gln Cys Met Gly Cys Cys Phe Ser Arg Ala Tyr
               525                 530                 535
   CCC ACT CCA CTA AGG TCC AAG AAG ACG ATG TTG GTC CAA AAG AAC GTC       1015
   Pro Thr Pro Leu Arg Ser Lys Lys Thr Met Leu Val Gln Lys Asn Val
           540                 545                 550
   ACC TCA GAG TCC ACT TGC TGT GTA GCT AAA TCA TAT AAC AGG GTC ACA       1063
   Thr Ser Glu Ser Thr Cys Cys Val Ala Lys Ser Tyr Asn Arg Val Thr
       555                 560                 565
   GTA ATG GGG GGT TTC AAA GTG GAG AAC CAC ACG GCG TGC CAC TGC AGT       1111
   Val Met Gly Gly Phe Lys Val Glu Asn His Thr Ala Cys His Cys Ser
   570                 575                 580                 585
   ACT TGT TAT TAT CAC AAA TCT TAAGGATCCC TCGAG                          1147
   Thr Cys Tyr Tyr His Lys Ser
                   590
(2) INFORMATION FOR SEQ ID NO:6:
   (i) SEQUENCE CHARACTERISTICS:
      (A) LENGTH: 285 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Ser Arg Thr Ser Leu Leu Leu Ala Phe Gly Leu Leu Cys Leu Pro Trp
   1               5                   10                  15
   Leu Gln Glu Gly Ser Ala Asp Ser Val Cys Pro Gln Gly Lys Tyr Ile
               20                  25                  30
   His Pro Gln Asn Asn Ser Ile Cys Cys Thr Lys Cys His Lys Gly Thr
           35                  40                  45
   Tyr Leu Tyr Asn Asp Cys Pro Gly Pro Gly Gln Asp Thr Asp Cys Arg
       50                  55                  60
   Glu Cys Glu Ser Gly Ser Phe Thr Ala Ser Glu Asn His Leu Arg His
   65                  70                  75                  80
   Cys Leu Ser Cys Ser Lys Cys Arg Lys Glu Met Gly Gln Val Glu Ile
                   85                  90                  95
   Ser Ser Cys Thr Val Asp Arg Asp Thr Val Cys Gly Cys Arg Lys Asn
               100                 105                 110
   Gln Tyr Arg His Tyr Trp Ser Glu Asn Leu Phe Gln Cys Phe Asn Cys
           115                 120                 125
   Thr Leu Cys Leu Asn Gly Thr Val His Leu Ser Cys Gln Glu Lys Gln
       130                 135                 140
   Asn Thr Val Cys Thr Cys His Ala Gly Phe Phe Leu Arg Glu Asn Glu
   145                 150                 155                 160
   Cys Val Ser Cys Ser Asn Cys Lys Lys Ser Leu Glu Cys Thr Lys Leu
                   165                 170                 175
   Ser Leu Pro Gln Ile Glu Asn Val Lys Gly Thr Glu Asp Ser Gly Thr
               180                 185                 190
   Thr Ala Gly Ala Ala Pro Gly Cys Pro Glu Cys Thr Leu Gln Glu Asn
           195                 200                 205
   Pro Phe Phe Ser Gln Pro Gly Ala Pro Ile Leu Gln Cys Met Gly Cys
       210                 215                 220
   Cys Phe Ser Arg Ala Tyr Pro Thr Pro Leu Arg Ser Lys Lys Thr Met
   225                 230                 235                 240
   Leu Val Gln Lys Asn Val Thr Ser Glu Ser Thr Cys Cys Val Ala Lys
                   245                 250                 255
   Ser Tyr Asn Arg Val Thr Val Met Gly Gly Phe Lys Val Glu Asn His
               260                 265                 270
   Thr Ala Cys His Cys Ser Thr Cys Tyr Tyr His Lys Ser
           275                 280                 285
(2) INFORMATION FOR SEQ ID NO:7:
   (i) SEQUENCE CHARACTERISTICS:
      (A) LENGTH: 1301 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 279..1287
   (xi) SEQUENCE DESCRIPTION: SEQ ID NO:7:
   CTCGAGATGG CTACAGGTAA GCGCCCCTAA AATCCCTTTG GGCACAATGT GTCCTGAGGG        60
   GAGAGGCAGC GACCTGTAGA TGGGACGGGG GCACTAACCC TCAGGTTTGG GGCTTCTGAA       120
   TGTGAGTATC GCCATGTAAG CCCAGTATTT GGCCAATGTC AGAAAGCTCC TGGTCCCTGG       180
   AGGGATGGAG AGAGAAAAAC AAACACCTCC TGGAGCAGGG AGAGTGCTGC CCTCTTGCTC       240
   TCCGGCTCCC TCTGTTGCCC TCTGGTTTCT CCCCAGGC TCC CGG ACG TCC CTG           293
   CTC CTG GCT TTT GGC CTG CTC TGC CTG CCC TGG CTT CAA GAG GGC AGT         341
   GCC GAT AGT GTG TGT CCC CAA GGA AAA TAT ATC CAC CCT CAA AAT AAT         389
   TCG ATT TGC TGT ACC AAG TGC CAC AAA GGA ACC TAC TTG TAC AAT GAC         437
   TGT CCA GGC CCG GGG CAG GAT ACG GAC TGC AGG GAG TGT GAG AGC GGC         485
   TCC TTC ACC GCT TCA GAA AAC CAC CTC AGA CAC TGC CTC AGC TGC TCC      533
   AAA TGC CGA AAG GAA ATG GGT CAG GTG GAG ATC TCT TCT TGC ACA GTG      581
   GAC CGG GAC ACC GTG TGT GGC TGC AGG AAG AAC CAG TAC CGG CAT TAT      629
   TGG AGT GAA AAC CTT TTC CAG TGC TTC AAT TGC AGC CTC TGC CTC AAT      677
   GGG ACC GTG CAC CTC TCC TGC CAG GAG AAA CAG AAC ACC GTG TGC ACC      725
   TGC CAT GCA GGT TTC TTT CTA AGA GAA AAC GAG TGT GTC TCC TGT AGT      773
   AAC TGT AAG AAA AGC CTG GAG TGC ACG AAG TTG TGC CTA CCC CAG ATT      821
   GAG AAT GTT AAG GGC ACT GAG GAC TCA GGC ACC ACA GCT GGT GCT GGT      869
   CCA CGG TGC CGC CCC ATC AAT GCC ACC CTG GCT GTG GAG AAG GAG GGC      917
   TGC CCC GTG TGC ATC ACC GTC AAC ACC ACC ATC TGT GCC GGC TAC TGC      965
   CCC ACC ATG ACC CGC GTG CTG CAG GGG GTC CTG CCG GCC CTG CCT CAG     1013
   GTG GTG TGC AAC TAC CGC GAT GTG CGC TTC GAG TCC ATC CGG CTC CCT     1061
   GGC TGC CCG CGC GGC GTG AAC CCC GTG GTC TCC TAC GCC GTG GCT CTC     1109
   AGC TGT CAA TGT GCA CTC TGC CGC CGC AGC ACC ACT GAC TGC GGG GGT     1157
   CCC AAG GAC CAC CCC TTG ACC TGT GAT GAC CCC CGC TTC CAG GAC TCC     1205
   TCT TCC TCA AAG GCC CCT CCC CCC AGC CTT CCA AGC CCA TCC CGA CTC     1253
   CCG GGG CCC TCG GAC ACC CCG ATC CTC CCA CAA T AAGGATCCCT CGAG       1301
(2) INFORMATION FOR SEQ ID NO:8:
   (i) SEQUENCE CHARACTERISTICS:
      (A) LENGTH: 336 amino acids
      (B) TYPE: amino acid
      (D) TOPOLOGY: linear
   (ii) MOLECULE TYPE: protein
   (xi) SEQUENCE DESCRIPTION: SEQ ID NO:8:
(2) INFORMATION FOR SEQ ID NO:9:
   (i) SEQUENCE CHARACTERISTICS:
      (A) LENGTH: 6 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:9:
   Ala Gly Ala Ala Pro Gly
   1 5
(2) INFORMATION FOR SEQ ID NO:10:
   (i) SEQUENCE CHARACTERISTICS:
      (A) LENGTH: 4 amino acids
      (B) TYPE: amino acid
      (C) STRANDEDNESS : single
      (D) TOPOLOGY: linear
   (ii) MOLECULE TYPE: peptide
   (xi) SEQUENCE DESCRIPTION: SEQ ID NO:10:
   Ala Gly Ala Gly
   1
(2) INFORMATION FOR SEQ ID NO:11:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:11:
      TTTTCTCGAG ATGGCTACAG GTAAGCGCCC 30
(2) INFORMATION FOR SEQ ID NO:12:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:12:
      ACCTGGGGCA GCACCGGCAC AGGAGACACA CTCGTTTTC 39
(2) INFORMATION FOR SEQ ID NO:13:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:13:
      TGTGCCGGTG CTGCCCCAGG TTGCCCAGAA TGCACGCTAC AG 42
(2) INFORMATION FOR SEQ ID NO:14:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:14:
      TTTTGGATCC TTAAGATTTG TGATAATAAC AAGTAC 36
(2) INFORMATION FOR SEQ ID NO:15:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:15:
      CCGTGGACCA GCACCAGCAC AGGAGACACA CTCGTTTTC 39
(2) INFORMATION FOR SEQ ID NO:16:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION SEQ ID NO:16:
      TGTGCTGGTG CTGGTCCACG GTGCCGCCCC ATCAAT 36
(2) INFORMATION FOR SEQ ID NO:17:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:17:
      TTTTGGATCC TTATTGTGGG AGGATCGGGG TG 32
(2) INFORMATION FOR SEQ ID NO:18:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:18:
      TTTTAGATCT CTTCTTGCAC AGTGGAC 27
(2) INFORMATION FOR SEQ ID NO:19:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:19:
      TGTGGTGCCT GAGTCCTCAG T 21
(2) INFORMATION FOR SEQ ID NO:20:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:20:
      ACTGAGGACT CAGGCACCAC AGCCGGTGCT GCCCCAGGTT G 41
(2) INFORMATION FOR SEQ ID NO:21:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:21:
      TTTTTCTAGA GAAGCAGCAG CAGCCCATG 29
(2) INFORMATION FOR SEQ ID NO:22:
   (i) SEQUENCE CHARACTERISTICS,
      (A) LENGTH: 75 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:22:
   TTTTCCACAG CCAGGGTGGC ATTGATGGGG CGGCACCGTG GACCAGCACC AGCTGTGGTG 60
   CCTGAGTCCT CAGTG 75

## Description

### FIELD OF THE INVENTION

The present invention relates to a hybrid protein comprising two coexpressed amino acid sequences forming a dimer, each comprising:
a) at least one amino acid sequence selected from the group consisting of antibody light chains; antibody heavy chains; and antibody Fab domains; and
b) a subunit of a heterodimeric proteinaceous hormone or fragments thereof which retain the ability of the subunit to form a heterodimer with other subunits thereof; wherein sequences (a) and (b) are bonded directly or through a peptide linker, and, wherein in each couple, the two subunits (b) are different and capable of aggregating to form a dimer complex.

### BACKGROUND OF THE INVENTION

Protein-protein interactions are essential to the normal physiological functions of cells and multicellular organisms. Many proteins in nature exhibit novel or optimal functions when complexed with one or more other protein chains. This is illustrated by various ligand-receptor combinations that contribute to regulation of cellular activity. Certain ligands, such as tumor necrosis factor a (TNFα), TNFβ, or human chorionic gonadotropin (hCG), occur as multi-subunit completes. Some of these complexes contain multiple copies of the same subunit. TNFα and TNFβ (collectively referred to hereafter as TNF) are homotrimers formed by three identical subunits (1-4). Other ligands are composed of non-identical subunits. For example, hCG is a heterodimer (5-7). Receptors may also occur or function as multi-chain complexes. For example, receptors for TNF transduce a signal after being aggregated to form dimers (8,9). Ligands to these receptors promote aggregation of two or three receptor chains, thereby affording a mechanism of receptor activation. For example, TNF-mediated aggregation activates TNF receptors (10-12).

The modulation of protein-protein interactions can be a useful mechanism for therapeutic intervention in various diseases and pathologies. Soluble binding proteins, that can interact with ligands, can potentially sequester the ligand away from the receptor, thereby reducing the activation of that particular receptor pathway. Alternatively, sequestration of the ligand may delay its elimination or degradation, thereby increasing its duration of effect, and perhaps its apparent activity *in vivo*. In the case of TNF, soluble TNF receptors have been primarily associated with inhibition of TNF activity (13-17).

Soluble binding proteins may be useful for treating human diseases. For example, soluble TNF receptors have been shown to have efficacy in animal models of arthritis (18,19).

Since TNF has three binding sites for its receptor (10-12), and dimerization of the cell surface receptor is sufficient for bioactivity (8,9), it is likely that binding of a single soluble receptor to TNF will leave open the possibility that this 1:3 complex of soluble receptor:TNF (trimer) can still bind and activate a pair of cell surface TNF receptors. To achieve an inhibitory effect, it would be expected that two of the receptor binding sites on the TNF trimer must be occupied or blocked by the soluble binding protein. Alternatively, the binding protein could block proper orientation of TNF at the cell surface.

Generally speaking, the need was felt of synthesizing proteins that contain two receptor (or ligands) chains, as dimeric hybrid protein. See Wallach et al., U.S. patent 5,478,925.

The primary strategy employed for generating dimeric or multimeric hybrid proteins, containing binding domains from extracellular receptors, has been to fuse these proteins to the constant regions of an antibody heavy chain.

This strategy led, for example, to the construction of CD4 immunoadhesins (20). These are hybrid molecules consisting of the first two (or all four) immunoglobulin-like domains of CD4 fused to the constant region of antibody heavy and light chains. This strategy for creating hybrid molecules was adapted to the receptors for TNF (10,16,21) and led to the generation of constructs with higher *in vitro* activity than the monomeric soluble binding proteins.

It is widely held that the higher *in vitro* potency of the dimeric fusion proteins should translate into higher *in vivo* activity. One study does support this, revealing an at least 50-fold higher activity for a p75 (TBP2)-Ig fusion protein in protecting mice from the consequences of intravenous LPS injection (16).

However, despite the widespread utilization of immunoglobulin fusion proteins, this strategy has several drawbacks. One is that certain immunoglobulin Fc domains participate in effector functions of the immune system. These functions may be undesirable in a particular therapeutic setting (22).

A second limitation pertains to the special cases where it is desirable to produce heteromeric fusion proteins, for example soluble analogs of the heteromeric IL-6 or type I interferon receptors. Although there are numerous methods for producing bifunctional antibodies (e.g., by co-transfection or hybridoma fusions), the efficiency of synthesis is greatly compromised by the mixture of homodimers and heterodimers that typically results (23). Recently there have been several reports describing the use of leucine zipper motifs to guide assembly of heterodimers (24-26). This appears to be a promising approach for research purposes, but the non-native or intracellular sequences employed may not be suitable for chronic applications in the clinic due to antigenicity. The efficiency of assembly and stability post assembly may also be limitations.

On the other hand, in the particular case of TNF receptors, certain modifications to the p55 TNF receptor have been found to facilitate homodimerization and signaling in the absence of ligand (27,28). It has been found that a cytoplasmic region of the receptor, termed the "death domain," can act as a homodimerization motif (28,30). As an alternative to an immunoglobulin hybrid protein, fusion of the extracellular domain of the TNF receptor to its cytoplasmic death domain could conceivably result in a secreted protein which can dimerize in the absence of TNF. Such fusion proteins have been disclosed and claimed in the International Patent Application WO 95/31544.

A third further strategy employed for generating dimers of soluble TNF receptors has been to chemically crosslink the monomeric proteins with polyethylene glycol (31).

### SUMMARY OF THE INVENTION

An alternative for obtaining such dimeric proteins, offering some important advantages, is the one of the present invention and consists in using a natural heterodimeric scaffold corresponding to a circulating non-immunoglobulin protein with a long half-life. A preferred example is hCG, a protein that is secreted well, has good stability, and has a long half-life (32-33). Given hCG's prominent role as a marker of pregnancy, many reagents have been developed to quantitate and study the protein *in vitro* and *in vivo*. In addition, hCG has been extensively studied using mutagenesis, and it is known that small deletions to the protein, such as removal of five residues at the extreme carboxyl-terminus of the α subunit, can effectively eliminate its biological activity while preserving its capability to form heterodimer (34,35). Small insertions, of up to 30 amino acids, have been shown to be tolerated at the amino- and carboxyl-termini of the α subunit (36), while fusion of the α subunit to the carboxyl terminus of the β subunit also had little effect on heterodimer formation (37).

An analog of hCG in which an immunoglobulin Fc domain was fused to the C-terminus of hCG β subunit has also been reported; however, this construct was not secreted and no effort was made to combine it with an α subunit (38).

Therefore, the main object of the present invention is a hybrid protein comprising two coexpressed amino acid sequences forming a dimer, each comprising:
a) at least one amino acid sequence selected from the group consisting of antibody light chains, antibody heavy chains; and antibody Fab domains; and
b) a subunit of a heterodimeric proteinaceous hormone, or fragments thereof which retain the ability of the subunit to form a heterodimer with other subunits thereof; in which sequences (a) and (b) are bonded directly or through a peptide linker, and wherein in each couple the two subunits (b) are different and capable of aggregating forming a dimer complex.

According to the present invention, the linker may be enzymatically cleavable.
Sequence (a) is preferably selected among:
   antibody light chains, antibody heavy chains or antibody Fab domains.
Sequence (b) is preferably selected among a hCG, FSH, LH, TSH, inhibin subunit, or fragments thereof.

Modifications to the proteins, such as chemical or protease cleavage of the protein backbone, or chemical or enzymatic modification of certain amino acid side chains, can be used to render the components of the hybrid protein of the invention inactive. This restriction of activity may also be accomplished through the use of recombinant DNA techniques to alter the coding sequence for the hybrid protein in a way that results directly in the restriction of activity to one component, or that renders the protein more amenable to subsequent chemical or enzymatic modification.

The above hybrid proteins will result in monofunctional, bifunctional or multifunctional molecules, depending on the amino acid sequences (a) that are combined with (b). In each couple, (a) can be linked to the amino termini or to the carboxy termini of (b), or to both.

A monoclonal hybrid protein of the present disclosure can, for instance, comprise the extracellular domain of a gonadotropin receptor linked to one of the corresponding receptor-binding gonadotropin subunits. According to such an embodiment, the hybrid protein can be a molecule in which, for example, the FSH receptor extracellular domain is linked to FSH to increase plasma half-life and improve biological activity.

This preparation can be employed to induce follicular maturation in assisted reproduction methods, such as ovulation induction or in vitro fertilisation, and to serve as a means to dramatically amplify the biological activity of the hormone essential for the success of the process, thus reducing the requirement for both the hormone itself and the number of injections to achieve ovulation.

The FSH receptor and the production of the extracellular domain of the human FSH receptor have been described respectively in WO 92/16620 and WO 96/38575.

The extracellular domain of the FSH receptor (ECD) can be fused in frame with a peptide linker that contains the thrombin recognition/cleavage site (29) and represents a "tethered" arm. The peptide linker links the extracellular domain of FSH with a FSH subunit. This will allow for removal of the extracellular domain of the FSH receptor by cleavage at the thrombin cleavage site as the molecule comes in contact with thrombin in the systemic circulation.

Instead of the thrombin cleavage site, an enzyme recognition site for an enzyme, that is found in greatest abundance in the ovary is used. In this way, as the ECD-FSH molecule travels to the ovary, it will be exposed to enzymes found in the highest concentrations in that tissue and the ECD will be removed so that the FSH can interact with the membrane bound receptor.

Instead of an enzyme recognition site, a flexible hinge region is cloned between ECD and FSH so that the ECD will not be enzymatically removed from the hormone. In this way, when the ECD-FSH molecule arrives at the ovary, a competition will be established between the hinge-attached ECD and the ECD of the FSH receptor found on the ovarian cell membrane.

Also described is the hybrid protein consisting of the aggregation between a couple of aa sequences, one of which contains TBP1 (or the fragments from aa 20 to aa 161 or to aa 190) as (a) and the α subunit of hCG as (b), and the other contains always TBP1 (or the same fragments as above) as (a) and the β subunit of hCG, or fragments thereof, as (b).
Depending on the particular sequence that is chosen as (b) (the entire β subunit of hCG, or fragments or modifications thereof), the resulting hybrid protein will have one activity (only that of TBP1) or a combination of activities (that of TBP1 with that of hCG). In this latter case the hybrid protein can be used, for example, in the combined treatment of Kaposi's sarcoma and metabolic wasting in AIDS.

In a preferred embodiment of the invention, one or more covalent bonds between the two subunits (b) are added to enhance the stability of.the resulting hybrid protein. This can be done, e.g., by adding one or more non-native interchain disulfide bonds. The sites for these cross-links can be deduced from the known structures of the heterodimeric hormones. For example, a suitable site in hCG could be to place cysteine residues at a subunit residue Lys45 and β subunit residue Glu21, replacing a salt bridge (non-covalent bond) with a disufide bond (covalent bond). Another object of the present invention are PEGylated or other chemically modified forms of the hybrid proteins.

A further object of the present invention is a DNA molecule comprising the DNA sequence coding for the above hybrid protein, as well as nucleotide sequences substantially the same. "Nucleotide sequences substantially the same" includes all other nucleic acid sequences which, by virtue of the degeneracy of the genetic code, also code for the given amino acid sequence.

For the production of the hybrid protein of the invention, the DNA sequence (a) is obtained from existing clones, as is (b). The DNA sequence coding for the desired sequence (a) is ligated with the DNA sequence coding for the desired sequence (b). Two of these fused products are inserted and ligated into a suitable plasmid or each into a different plasmid. Once formed, the expression vector, or the two expression vectors, is introduced into a suitable host cell, which then expresses the vector(s) to yield the hybrid protein of the invention as defined above.

The preferred method for preparing the hybrid of the invention is by way of PCR technology using oligonucleotides specific for the desired sequences to be copied from the clones encoding sequences (a) and (b).

Expression of any of the recombinant proteins of the invention as mentioned herein can be effected in eukaryotic cells (e.g., yeasts, insect or mammalian cells) or prokaryotic cells, using the appropriate expression vectors. Any method known in the art can be employed.

For example the DNA molecules coding for the proteins obtained by any of the above methods are inserted into appropriately constructed expression vectors by techniques well known in the art (see Sambrook et al, 1989). Double stranded cDNA is linked to plasmid vectors by homopolymeric tailing or by restriction linking involving the use of synthetic DNA linkers or blunt-ended ligation techniques: DNA ligases are used to ligate the DNA molecules and undesirable joining is avoided by treatment with alkaline phosphatase.

In order to be capable of expressing the desired protein, an expression vector should comprise also specific nucleotide sequences containing transcriptional and translational regulatory information linked to the DNA coding the desired protein in such a way as to permit gene expression and production of the protein. First in order for the gene to be transcribed, it must be preceded by a promoter recognizable by RNA polymerase, to which the polymerase binds and thus initiates the transcription process. There are a variety of such promoters in use, which work with different efficiencies (strong and weak promoters).

For eukaryotic hosts, different transcriptional and translational regulatory sequences may be.employed, depending on the nature of the host. They may be derived form viral sources, such as adenovirus, bovine papilloma virus, Simian virus or the like, where the regulatory signals are associated with a particular gene which has a high level of expression. Examples are the TK promoter of the Herpes virus, the SV40 early promoter, the yeast gal4 gene promoter, etc. Transcriptional initiation regulatory signals may be selected which allow for repression and activation, so that expression of the genes can be modulated.

The DNA molecule comprising the nucleotide sequence coding for the hybrid protein of the invention is inserted into a vector(s), having the operably linked transcriptional and translational regulatory signals, which is capable of integrating the desired gene sequences into the host cell. The cells which have been stably transformed by the introduced DNA can be selected by also introducing one or more markers which allow for selection of host cells which contain the expression vector. The marker may also provide for phototrophy to a auxotropic host, biocide resistance, e.g., antibiotics, or heavy metals such as copper, or the like. The selectable marker gene can either be directly linked to the DNA gene sequences to be expressed, or introduced into the same cell by co-transfection. Additional elements may also be needed for optimal synthesis of proteins of the invention.

Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species.

Once the vector(s) or DNA sequence containing the construct(s) has been prepared for expression, the DNA construct(s) may be introduced into an appropriate host cell by any of a variety of suitable means: transformation, transfection, conjugation, protoplast fusion, electroporation, calcium phosphate-precipitation, direct microinjection, etc.

Host cells may be either prokaryotic or eukaryotic. Preferred are eukaryotic hosts, e.g., mammalian cells, such as human, monkey, mouse, and Chinese hamster ovary (CHO) cells, because they provide post-translational modifications to protein molecules, including correct folding or glycosylation at correct sites. Also, yeast cells can carry out post-translational peptide modifications including glycosylation. A number of recombinant DNA strategies exist which utilize strong promoter sequences and high copy number of plasmids which can be utilized for production of the desired proteins in yeast. Yeast recognizes leader sequences on cloned mammalian gene products and secretes peptides bearing leader sequences (i.e., pre-peptides).

After the introduction of the vector(s), the host cells are grown in a selective medium, which selects for the growth of vector-containing cells. Expression of the cloned gene sequence(s) results in the production of the desired proteins.

Purification of the recombinant proteins is carried out by any one of the methods known for this purpose, i.e., any conventional procedure involving extraction, precipitation, chromatography, electrophoresis, or the like. A further purification procedure that may be used in preference for purifying the protein of the invention is affinity chromatography using monoclonal antibodies which bind the target protein and which are produced and immobilized on a gel matrix contained within a column. Impure preparations containing the recombinant protein are passed through the column. The protein will be bound to the column by the specific antibody while the impurities will pass through. After washing, the protein is eluted from the gel by a change in pH or ionic strength.

The term "hybrid protein", as used herein, generically refers to a protein which contains two or more different proteins or fragments thereof.

As used herein, "fusion protein" refers to a hybrid proteins, which consists of two or more proteins, or fragments thereof, linked together covalently.

The term "aggregation", as used herein, means the formation of strong specific non-covalent interactions between two polypeptide chains forming a complex, such as those existing between the α any β subunit of a heterodimeric hormone (such as FSH, LH, hCG or TSH).

The terms "ligand" or "ligand protein", as used herein, refer to a molecule, other than an antibody or an immunoglobulin, capable of being bound by the ligand-binding domain of a receptor; such molecule may occur in nature, or may be chemically modified or chemically synthesised.

The term "ligand-binding domain" as used herein, refers to a portion of the receptor that is involved in binding a ligand and is generally a portion or essentially all of the extracellular domain.

The term "receptor", as used herein, refers to a membrane protein, whose binding with the respective ligand triggers secondary cellular responses that result in the activation or inhibition of intracellular process.

In a further aspect, the present invention provides the use of the hybrid protein as a medicament. The medicament is preferably presented in the form of a pharmaceutical composition comprising the protein of the invention together with one or more pharmaceutically acceptable carriers and/or excipient. Such pharmaceutical compositions represent yet a further aspect of the invention.

Also disclosed is:
1. A hybrid protein comprising two coexpressed amino acid sequences forming a dimer, each comprising:
   a) at least one amino acid sequence selected from the group consisting of a homomeric receptor, a chain of a heteromeric receptor, a ligand, and fragments thereof which retain the ligand-receptor binding capability; and
   b) a subunit of a heterodimeric proteinaceous hormone, or fragments thereof which retain the ability of the subunit to form a heterodimer with other subunits thereof;
      wherein sequences (a) and (b) are bonded directly or through a peptide linker, and in which the sequence (b) in each of said two coexpressed sequences are capable of aggregating to form a dimer complex.
2. A hybrid protein in accordance with item 1,
   wherein said sequence (a) is selected from the group consisting of TBP1, TBP2 or fragments thereof still containing the ligand binding domain; the extracellular domain of the IFNα/β receptor or the IFNγ receptor; a gonadotropin receptor or extracellular fragments thereof; antibody light chains or fragments thereof, optionally associated with the respective heavy chains; antibody heavy chains or fragments thereof; antibody Fab domain; and IL-6, IFN-β, TPO or fragments thereof.
3. A hybrid protein in accordance with item 1, wherein said sequence (b) is selected from the group consisting of subunits of hCG, FSH, LH, TSH or inhibin, and fragments thereof.
4. A hybrid protein in accordance with item 1, wherein sequence (a) is linked to the amino terminus of sequence (b).
5. A hybrid protein in accordance with item 1,
   wherein sequence (a) is linked to the carboxy terminus of sequence (b).
6. A hybrid protein in accordance with item 1,
   wherein said two coexpressed amino acid sequences each include the sequence for TBP1 or the fragment thereof corresponding to amino acid residues 20-161 or 20-190 of TBP1, as sequence (a) and the respective α and β subunits of hCG or fragments thereof, as sequence (b).
7. A hybrid protein in accordance with item 1,
   wherein said two coexpressed amino acid sequences each include the extracellular domain of a gonadotropin receptor as sequence (a) and the respective a and β subunits of a gonadotropin as sequence (b).
8. A hybrid protein in accordance with item 7,
   wherein said sequence (a) is the FSH receptor extracellular domain and sequence (b) is a subunit of FSH.
9. A hybrid protein in accordance with item 7,
   wherein said sequences (a) and (b) are linked with a peptide linker.
10. A hybrid protein in accordance with item 9,
   wherein said peptide linker has an enzyme cleavage site.
11. A hybrid protein in accordance with item 10,
   wherein said enzyme cleavage site is a thrombin cleavage site.
12. A hybrid protein in accordance with item 10,
   wherein said enzyme cleavage site is recognized and cleaved by an enzyme which is found in the ovary.
13. A hybrid protein in accordance with item 9,
   wherein said peptide linker serves as a flexible hinge.
14. A hybrid protein in accordance with item 1,
   wherein one or more covalent bonds between the two subunits (b) are added.
15. A DNA molecule encoding a hybrid protein in accordance with item 1.
16. An expression vector containing a DNA molecule in accordance with item 15.
17. A host cell containing an expression vector in accordance with item 16 and capable of expressing said hybrid protein.
18. A method for producing hybrid protein comprising culturing a host cell in accordance with item 17 and recovering the hybrid protein expressed thereby.
19. A pharmaceutical composition comprising a hybrid protein in accordance with item 1 and a pharmaceutically acceptable carrier and/or excipient.
20. A method for inducing follicular maturation, comprising administering a pharmaceutical composition comprising the hybrid protein of item 8 to a subject in need thereof.

### BRIEF DESCRIPTION OF THE DRAWINGS

The invention will be better understood by reference to the appended drawings, in which:
Figures 1(a) and 1(b) show the TBP(20-161)-hCGα and TBP(20-161)-hCGβ constructs, respectively, and the corresponding sequences (SEQ ID NOS:1-4).
Figures 2(a) and 2(b) show the TBP(20-190)-hCGα and TBP(20-190)-hCGβ constructs, respectively, and the corresponding sequences (SEQ ID NOS:5-8).
Figure 3 is a schematic summary of the constructs of Figure 3 is a schematic summary of the constructs of Figures 1 and 2 showing p55 TNFR1, TBP1 and TBP1 fusion contructs. The linker sequences shown on the last two lines are SEQ ID NO:9 (Ala-Gly-Ala-Ala-Pro-Gly) and SEQ ID NO:10 (Ala-Gly-Ala-Gly).
Figure 4 is a graph illustrating the dose dependent protective effect of CHO cell expressed TBP-hCG(20-190) on TNFα-induced cytotoxicity on BT-20 cells and various controls.
Figure 5 is a graph illustrating the dose dependent. protective effect of COS cell expressed TBP-hCG(20-190) on TNFα-induced cytotoxicity on BT-20 cells and various controls.
Figure 6 is a graph illustrating the dose dependent protective effect of affinity purified CHO cell expressed TBP-hCG(20-161) on TNFα-induced cytotoxicity on BT-20 cells and various controls.

### DETAILED DESCRIPTION OF THE PREFERRED EMBODIMENTS

The invention will now be described by means of the following Examples, which should not be construed as in any way limiting the present invention.

### EXAMPLES

### Materials and Methods

Cell lines used in this study were obtained from the American Type Culture Collection (ATCC), Rockville, Maryland, unless otherwise specified. The CHO-DUKX cell line was obtained from L. Chasin at Columbia University through D. Houseman at MIT (39). The CHO-DUKX cells, which lack a functional gene for dihydrofolate reductase, were routinely maintained in complete α-plus Modified Eagles Medium (α(+)MEM) supplemented with 10% fetal bovine serum (FBS). The COS-7 cells were routinely maintained in Dulbecco's Modified Eagles Medium (DMEM) supplemented with 10% FBS. Unless specified otherwise, cells were split to maintain them in log phase of growth, and culture reagents were obtained from GIBCO (Grand Island, New York).

### 1. Assembly of the genetic constructs encoding the hybrid proteins

The numbering assignments for the p55 TNF receptor are based on the cloning paper from Wallach (40), while the numbering assignments for the hCG subunits are based on the numbering assignments from the Fiddes cloning papers (41,42). The designation TBP, or TNF binding protein, refers to the extracellular domain portions of the TNF receptors capable of binding TNF. In these Examples, the DNA constructs will be named as TBP-hybrid proteins, with the partner and region of TBP indicated in the construct nomenclature. All of the TBP-hCG constructs contain the human growth hormone (hGH) signal peptide in place of the native p55 signal sequence. In addition, the hGH signal peptide has been placed so that it immediately precedes TBP residue Asp20, which is anticipated to make this the first residue in the mature, secreted protein. These modifications are not essential to the basic concept of using hCG as a partner of the hybrid protein.

The DNAs encoding the hybrid proteins were constructed using PCR methodology (43).

### a. TBP1(20-161)-hCG

The initial TBP-hCG construct was engineered to contain the ligand binding domain from the extracellular region of the p55 TNF receptor (from Asp20 inclusive of residue Cys161) fused though a short linker to the hCG α and β subunits (starting at residues αCys7 or βPro7, respectively). This construct, hereafter referred to as TBP1(20-161)-hCG, is a heterodimer of two modified hCG subunits, TBP1(20-161)-hCGα and TBP1(20-161)-hCGβ.

The oligodeoxynucleotide primers used for the TBP1(20-161)-hCGα construct were:

| | |
|---|---|
| primer 1(αβ) | |
| primer 2(α) | |
| primer 3(α) | |
| primer 4 (α) | |

These and all of the other primers described in these Examples were synthesized on an Applied Biosystems Model 392 DNA synthesis machine (ABI, Foster City, California), using phosphoramidite chemistry.

Since both of the TBP-hCG subunit constructs have the same 5'-end (i.e., the 5'-end of the hGH/TBP construct), primer 1(αβ) was used for both TBP-hCG subunit constructs. The other primers used for the TBP1(20-161)-hCGβ construct were:

| | |
|---|---|
| primer 2(β) | |
| primer 3(β) | |
| primer 4(β) | |

Primers 2(α) and 3(a) are reverse complements, and cover both the 3'-end of the coding region for the p55 extracellular domain, and the 5'-end of the hCG α subunit. Similarly, primers 2(β) and 3(β) are also reverse complements, and cover both the 3'-end of the coding region for the p55 extracellular domain, and the 5'-lend of the hCG β subunit.

Two PCR reactions were run for each of the two TBP-hCG subunit constructs. The first used primers 1(αβ) and 2 (α or β), and used as the template a plasmid encoding soluble p55 residues 20-180 preceded by the hGH signal peptide (plasmid pCMVhGHspcDNA.pA4). The second used primers 3 (α or β) and 4 (α or β), and used as the template either plasmid pSVL-hCGα or pSVL-hCGβ (44). The PCR was performed using Vent (TM) polymerase from New England Biolabs (Beverly, Massachusetts) in accordance with the manufacturer's recommendations, using for each reaction 25 cycles and the following conditions:
100 µg of template DNA
1 µg of each primer
2U of Vent(TM) polymerase (New England Biolabs) denaturation at 99°C for 30 seconds
annealing at: 59°C for 30 seconds for primers 1(αβ) and 2(α)
59°C for 30 seconds for primers 3(α) and 4(α)
57°C for 30 seconds for primers 1(αβ) and 2(β)
63°C for 30 seconds for primers 3(β) and 4(β) extension at 75°C for 75 seconds.

The PCR products were confirmed to be the expected size by electrophoresis in a 2% agarose gel and ethidium bromide staining. The fragments were then purified by passage over a Wizard column (Promega) in accordance with the column manufacturer's recommendations.

The final coding sequence for TBP1(20-161)-hCGα was assembled by fusion PCR using primer 1(αβ) and primer, 4(α), and using as template the purified products from the p55 and hCG α fragments obtained from the first PCR reactions. First the two templates, which due to the overlap between primers 2(a) and 3(α) could be denatured and annealed together, were passed through 10 cycles of PCR in the absence of any added primers. The conditions for these cycles were essentially the same as those used earlier, except that the annealing was done at 67°C and the extension was performed for 2 minutes. At the end of these 10 cycles, primers 1(αβ) and 4(α) were added, and another 10 cycles were performed. The conditions for this final set of reactions was the same as used earlier, except that an annealing temperature of 59°C was used, and the extension was performed for 75 seconds.

Analysis of the products of this reaction by electrophoresis in a 1% agarose gel confirmed that the expected fragment of about 1100bp was obtained. The reaction was passed over a Wizard column to purify the fragment, which was then digested with XbaI and BamHI and re-purified in a 0.7% low-melting point agarose gel. The purified fragment was subcloned into plasmid pSVL (Pharmacia), which had first been digested with XbaI and BamHI and gel purified on a 0.8% low-melting point agarose gel. Following ligation with T4 ligase, the mixture was used to transform AG1 *E. coli* and then plated onto LB/ampicillin plates for overnight culture at 37°C. Plasmid DNAs from ampicillin-resistant colonies were analyzed by digestion with XhoI and BamHI to confirm the presence of the insert (which is excised in this digest). Six clones were found to contain inserts, and one (clone 7) was selected for further advancement and designated pSVLTBPhCGα (containing TBP1(20-161)-hCGα). Dideoxy DNA sequencing (using Sequenase^{™} U.S. Biochemicals, Cleveland, Ohio) of the insert in this vector confirmed that the construct was correct, and that no undesired changes had been introduced.

The final coding sequence for TBP1(20-161)-hCGβ was assembled in a manner similar to that described for TBP1(20-161)-hCGα using fusion PCR and primers 1(αβ) and 4(β), and using as template the purified products from the p55 and hCG β fragments obtained from the first PCR reactions. The resulting pSVL plasmid containing the insert of interest was designated pSVLTBPhCGβ.

### b. TBP (20-190)-hCG

A second set of TBP-hCG proteins was prepared by modification of the TBP(20-161)-hCG constructs to produce an analog containing TBP spanning from Asp20 to Thr190, in place of the 20-161 region in the initial analog. This was done by replacing the fragment between the BglII and XbaI sites in plasmid pSVLTBPhCGα with a PCR fragment containing the change. This PCR fragment was generated using fusion PCR. The primers were:

| | |
|---|---|
| primer 1 | TTT TAG ATC TCT TCT TGC ACA GTG GAC (SEQ ID NO:18) |
| primer 2 | TGT GGT GCC TGA GTC CTC AGT (SEQ ID NO:19) |
| primer 3 | |
| primer 4 | TTT TTC TAG AGA AGC AGC AGC AGC CCA TG (SEQ ID NO:21) |

Primers 1 and 2 were used to generate the sequence coding the additional p55 residues from 161-190. The PCR reaction was performed essentially as described earlier, using 1 µg of each primer and pUC-p55 as template. Similarly, primers 3 and 4 were used to generate by PCR the linker between the 3'-end of the TBP-coding region, and the 5'-end of the hCG a subunit coding region, using as a template plasmid pSVLTBPhCGα. Products from these PCR reactions were confirmed to be the correct size (about 296 bp and 121 bp respectively) by polyacrylamide gel electrophoresis (PAGE) on an 8% gel, and were then purified using a Wizard column. The design of primers 2 and 3 was such that they contained a region of overlap, so that the two PCR products (from primers 1 ant 2, and from primers 3 and 4) could be annealed for fusion PCR with primers 1 and 4. Subsequent to the fusion reaction, the desired product of about 400 bp was confirmed and purified using a 1.5% agarose gel and a Wizard column. This DNA was then digested with BglII and XbaI, and ligated with BglII/XbaI-digested pSVLTBPhCGα. The presence of an insert in plasmids isolated from transformed AG1 E. coli was confirmed by digestion with BglII and XbaI. The new construct was designated pSVLTBP(20-190)-hCGα.

Similarly, plasmid pSVLTBPhCGβ was modified by substitution of the BglII-XcmI fragment. However, this was done by subcloning of a single PCR product, rather than with a fusion PCR product. Primers 1 and 2b (see below) were used with pUC-p55 as the template.

| | |
|---|---|
| primer 2b | |

The resulting PCR product (about 337bp) was confirmed and purified as described above, digested with BglII and XcmI, and then ligated into BglII/XbaI-digested pSVLTBPhCGβ. The presence of an insert in plasmids isolated from transformed AG1 E. coli was confirmed by digestion with BglII and XcmI. The new construct was designated pSVLTBP(20-190)-hCGβ.

The new constructs were subsequently confirmed by DNA sequencing.

In addition to producing these new pSVL-based plasmids, these constructs were also subcloned into other expression vectors likely to be more suitable for stable expression in CHO, particularly vector Dα, previously described as plasmid CLH3AXSV2DHFR (45). This was accomplished by converting a BamHI site flanking the inserts in the pSVL-based vectors to an XhoI site, and then excising the insert with XhoI and cloning it into XhoI digested Dα.

### 2. Transient and stable expression of the hybrid proteins

Transfections of COS-7 cells (ATCC CRL 1651, ref. 46) for transient expression of the TBP-hCG hybrid proteins were performed using electroporation (47). Exponentially growing COS-7 cells were removed by trypsinization, collected by gentle centrifugation (800 rpm, 4 minutes), washed with cold phosphate buffered saline (PBS), pH 7.3-7.4, and then repelleted by centrifugation. Cells were resuspended at a concentration of 5x10⁶ cells per 400 µl cold PBS and mixed with 10 µg of plasmid DNA in a prechilled 2 mm gap electroporation cuvette. For cotransfections, 5 µg of each plasmid were used. The cuvette and cells were chilled on ice for a further 10 minutes, and then subjected to electroporation using a BTX Model 600 instrument and conditions of 125 V, 950µF and R=8. Afterward the cells were set to cool on ice for 10 minutes, transferred to a 15 ml conical tube containing 9.5 ml complete medium (Dulbecco's modified Eagle's medium (DMEM) supplemented with 10% fetal bovine serum (FBS) and 1% L-glutamine) at room temperature, and left at room temperature for 5 minutes. After gentle mixing in the 15 ml tube, the entire contents was seeded onto two P100 plates and placed into a 37°C, 5% CO₂ incubator. After 18 hours the media was changed, and in some cases the new media contained only 1% or 0% FBS. After another 72 hours, the conditioned media was harvested, centrifuged to remove cells, and then stored frozen at -70°C.

Transfections of CHO-DUKX (CHO) cells for transient or stable expression were performed using calcium phosphate precipitation of DNA. Twenty-four hours prior to the transfection, exponentially growing CHO cells were plated onto 100 mm culture plates at a density of 7.5x10⁵ cells per plate. On the day of the transfection, 10 µg of plasmid DNA was brought to 0.5 ml in transfection buffer (see below), 31 µl of 2 M CaCl₂ were added, the DNA-CaCl₂ solution was mixed by vortexing, and left to stand at room temperature for 45 minutes. After this the media was aspirated from the plates, the DNA was added to the cells using a sterile plastic pipette, and the cells were left at room temperature for 20 minutes. At the end of this period, 5 ml of complete α(+)MEM containing 10% FBS was added to the plates, which were incubated at 37°C for 4-6 hours. The media was then aspirated off the plates, and the cells were subjected to a glycerol shock by incubating them with a solution of 15% glycerol in transfection buffer at 37°C for 3.5 minutes. After removal of the glycerol solution, the cells were washed twice with PBS, refed with 10 ml complete α(+)MEM, 10% FBS, and returned to the 37°C incubator. For stable transfections, after 48 hours the cells were split 1:10 and fed with selection medium (complete α-minus MEM (lacking nucleosides), 10% dialyzed FBS, and 0.02 µM methotrexate). Non-transfected (non-resistant) cells were typically eliminated in 3-4 weeks, leaving a population of transfected, methotrexate-resistant cells.

### 3. Quantitation of expression

Secretion of the hybrid proteins by transfected cells was assessed using a commercial assay kit for soluble p55 (R&D Systems; Minneapolis, Minnesota) in accordance with the manufacturer's instructions. This assay also provides an estimate of the hybrid protein levels in conditioned and processed media, which served as the basis for selecting doses to be used in the bioassay.

### 4. Assessment of heterodimer formation

To assess the ability of the TBP-hCG subunit fusions to combine and form heterodimers, a sandwich immunoassay using antibodies to the hCG subunits was performed. In this assay, a monoclonal antibody to the hCG β subunit is coated onto microtiter plates and used for analyte capture. The primary detection antibody is a goat polyclonal raised against the human TSH α subunit (#082422G - Biodesign International; Kennenbunkport, Maine), which is in turn detected using a horse radish peroxidase conjugated rabbit anti-goat polyclonal antibody (Cappel; Durham, North Carolina).

Several different anti-hCG β subunit antibodies were used in this work, all of which show no detectable cross-reactivity with the free α subunit. One of these antibodies (3/6) is used in the commercially available MAIAclone hCG assay kit (Biodata; Rome, Italy).

High-protein binding microtiter plates (Costar #3590) were coated with capture antibody by incubation (2 hours at 37°C) with 100 µl/well of a 5 µg/ml solution of antibody in coating buffer (PBS, pH 7.4, 0.1 mM Ca⁺⁺ , , 0.1 mM mg⁺⁺). After washing once with wash solution (PBS, pH 7.4 + 0.1% Tween 20) the plate is blocked by completely filling the wells (=400 µl/well) with blocking solution (3% bovine serum albumin (BSA; fraction V - A-4503 Sigma) in PBS, pH 7.4) and incubating for one hour at 37°C or overnight at 4°C. The plate is then washed twice with wash solution, and the reference and experimental samples, diluted in diluent (5 mg/ml BSA in PBS, pH 7.4) to yield a 100 µl volume, are added. After incubating the samples and the plate for two hours at 37°C, the plate is again twice washed with wash solution. The primary detection antibody, diluted 1:5000 in diluent, is added (100 µl/well) and incubated for one hour at 37°C. The secondary detection antibody (HRP conjugated rabbit anti-goat Ig), diluted 1:5000 in diluent, is added (100 µl/well) and after incubation for one hour at 37°C, the plate is washed three times with wash solution. One hundred µl of TMB substrate solution (Kirkegaard and Perry Laboratories) is added, the plate is incubated 20 minutes in the dark at room temperature, and then the enzymatic reaction is stopped by addition of 50 µl/well 0.3M H₂SO₄. The plate is then analyzed using a microtiter plate reader set for a wavelength of 450 nm.

### 5. Partial purification

To better quantitate the activities of these hybrid proteins, TBP-hCG hybrid proteins were partially purified by immunoaffinity chromatography. The antibody used was a monoclonal commercially available from R&D Systems (MAB #225). The column was CNBr-activated sepharose, charged with the antibody by following the manufacturer's. (Pharmacia) instructions.

Conditioned media was collected from confluent T-175 flasks of each line using daily harvests of 50 ml SFMII media (GIBCO), five harvests for each line. The collections were subjected to centrifugation (1000 RPM) to remove cellular debris. The material was then assayed for TBP content using the commercial immunoassay and concentrated (Centricon units by Amicon; Beverly, Massachusetts) so that the apparent TBP concentration was about 50 ng/ml.

Ten ml of the concentrated TBP-hCG (sample #18873) was brought to approximately 1 M NaCl by addition of NaCl and adjustment of the solution to a conductivity of approximately 85 mS/cm. This was passed through a 0.5 ml anti-TBP immunoaffinity column. The flow-through was collected and run through the column a second time. After this the column was washed with 1 M NaCl in PBS. The bound TBP(20-161)-hCG was collected after elution with 50 mM citric acid (pH 2.5). The eluate (approximately 7 ml) was concentrated by filtration using Amicon Centricon-10's in accordance with the manufacturer's (Amicon) instructions, to a volume of approximately 200 µl. Approximately 800 µl of PBS was added to bring the sample volume to 1 ml, which was stored at 4°C until tested by bioassay.

### 6. Assessment of anti-TNF activity

Numerous in vitro TNF-induced cytotoxicity assays have been described for evaluating analogs of soluble TNF receptors. We utilized an assay employing a human breast carcinoma cell line, BT-20 cells (ATCC HTB 19). The use of these cells as the basis for a TNF bioassay has been described previously (48). These cells are cultured at 37°C in RPMI 1640 media supplemented with 10% heat-inactivated FBS. The cells were grown to a maximum 80-90% confluence, which entailed splitting every 3-4 days with a seeding density of about 3x10⁶ cells per T175cm² flask.

The BT-20 assay uses the inclusion of a cellular stain, crystal violet, as a detection method to assess survival of cells after treatment with TNF. Dead cells are unable to take up and retain the dye.

In brief, the protocol used for the assay of anti-TNF activity is the following. Recombinant human TNFα (R&D Systems) and the experimental samples are constituted in media (RPMI 1640 with 5% heat-inactivated FBS) and added to the wells of 96-well culture plates. The cells are then plated into these wells at a density of 1x10⁵ cells/well. The quantity of TNFα added was determined earlier in titration studies, and represents a dose at which about 50% of the cells are killed.

After addition of the samples, the cells are cultured for 48 hours at 39°C, after which the proportion of live cells is determined using crystal violet staining and a microtiter plate reader (570 nm).

### RESULTS

### 1. Constructs under study

The designs of the hybrid proteins studied are briefly summarized below; two control proteins, a monomeric soluble p55 (r-hTBP-1) and a dimeric TBP-immunoglobulin fusion protein (TBP-IgG3) (prepared essentially as described in (10)), were studied for comparative purposes.

| Construct | TBP N-term | TBP C-term | Fusion partner |
|---|---|---|---|
| r-hTBP-1 | mix of 9 and 20 | 180 | none |
| TBP-IgG3 | mix of 9 and 20 | 190 | IgG3 heavy chain constant region |
| TBP (20-161) -hCG | 20 | 161 | hCG and hCG*β* (heterodimer) |
| TBP(20-190)-hCG | 20 | 190 | hCGα and hCG*β* (heterodimer) |

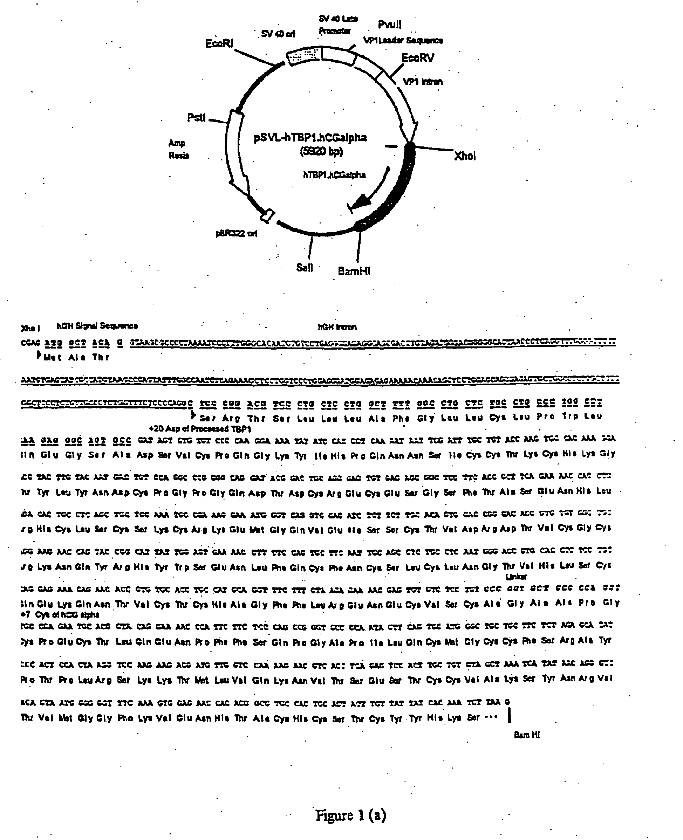

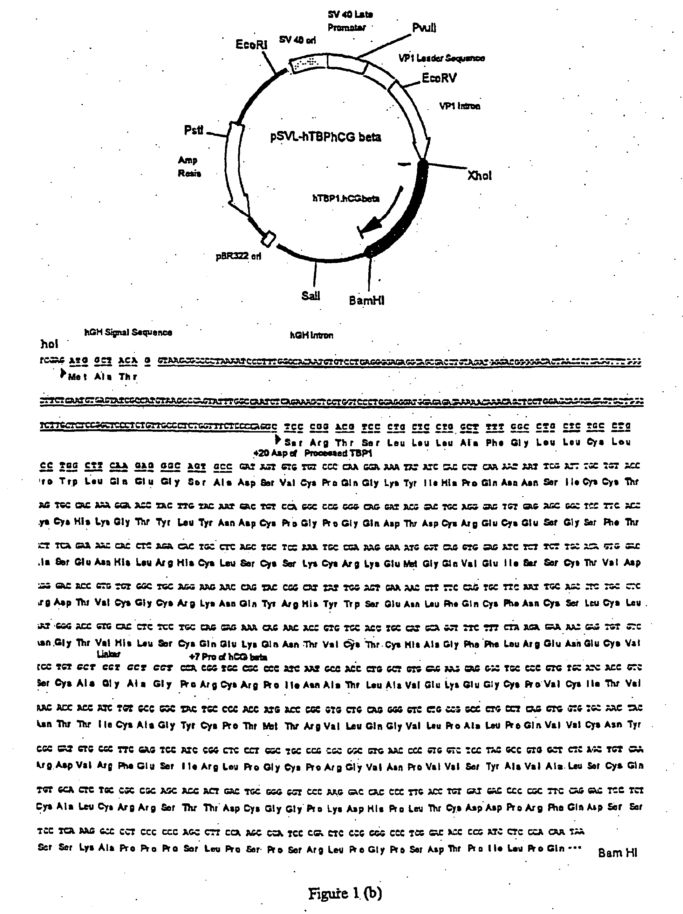

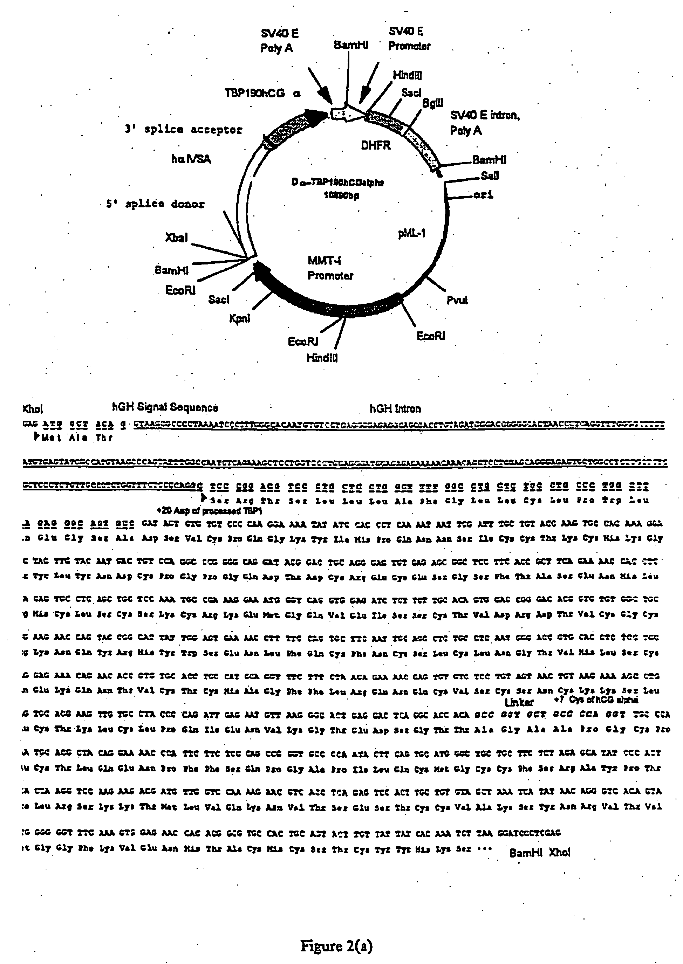

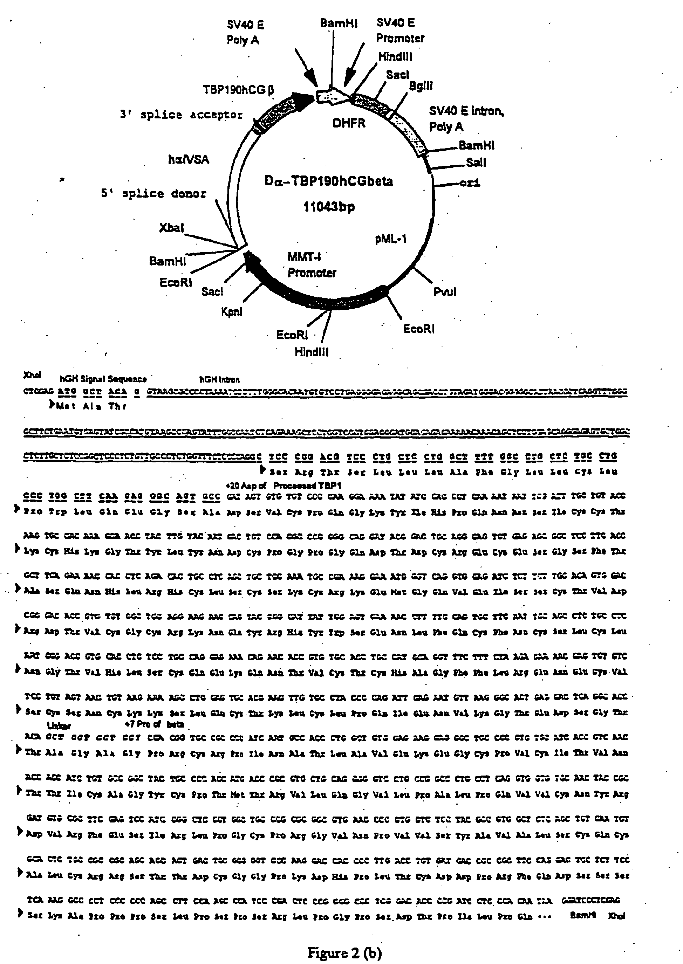

The sequences of the DNAs encoding, TBP(20-190)-hCG and TBP(20-161)-hCG are provided in Figures 1 and 2, respectively. A schematic summary of the constructs is provided in Figure 3.

### 2. Secretion of TBP-hCG proteins

All of the constructs tested were found to be produced and secreted into culture media by transfected mammalian cells. Data illustrating this are shown in Tables 1 and 2.

### 3. TBP-hCG(α/ß) fusion proteins assemble into heterodimers

The combination of TBP-hCGα and TBP-HCGß was confirmed using the sandwich assay for.the hCG heterodimer. Only the combined transfection of α and ß subunit fusions resulted in heterodimer detection (Table 3).

### 4. TBP-hCG hybrid proteins exhibit increased activity over TBP monomer

Hybrid proteins produced in either COS-7 or CHO cells were found to be potent, inhibitors of TNFα in the BT-20 bioassay. Some of the samples tested are summarized in Table 4.

Negative controls (conditioned media from mock transfections) were included for the 1x media samples.

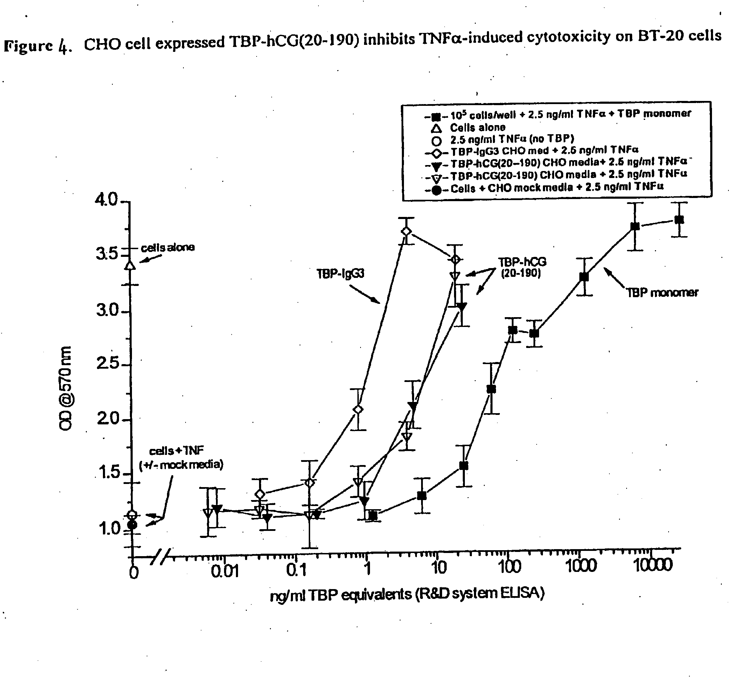

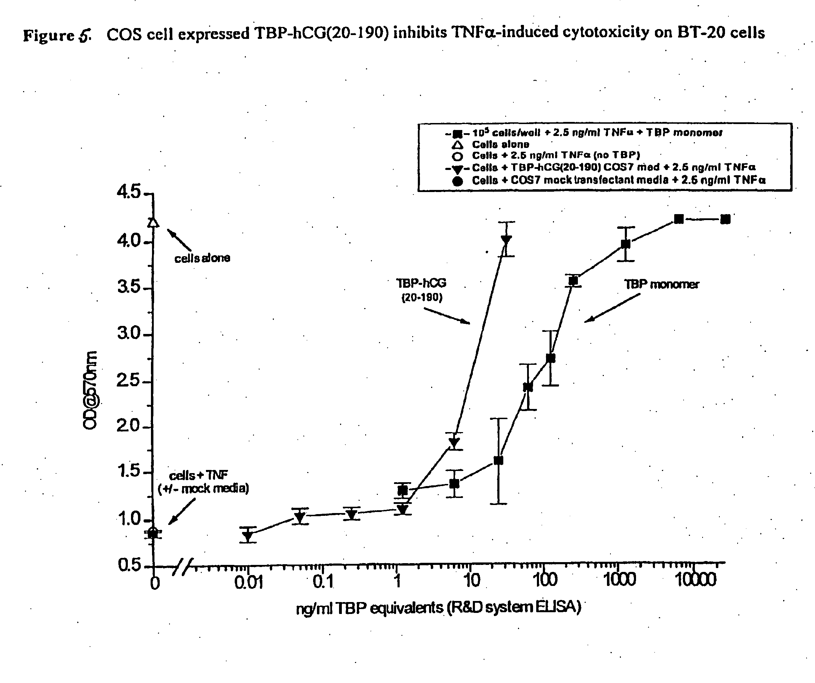

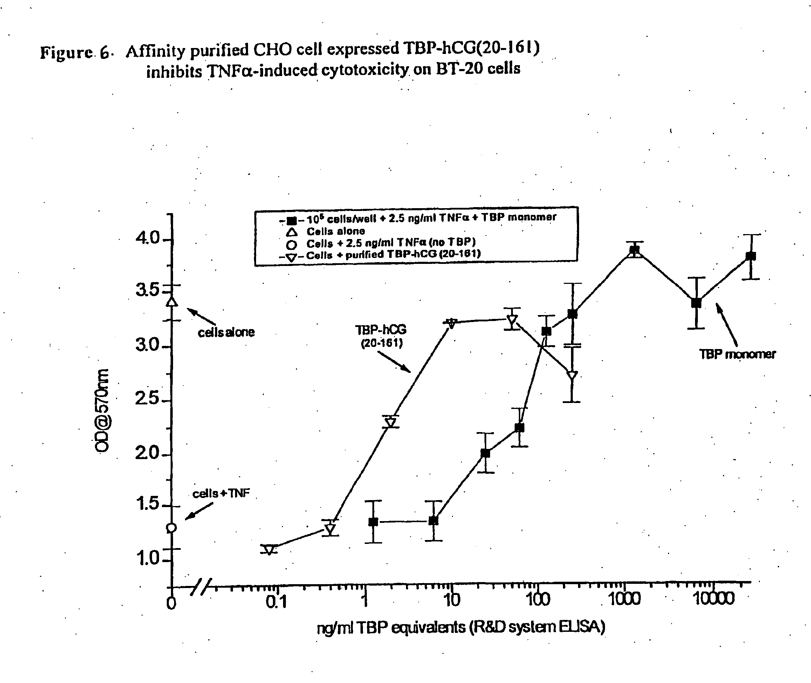

As illustrated in Figures 4-6 (points on γ-axis), addition of TNF (2.5 ng/ml) results in a clear reduction in live cell number (as assessed by OD 570). In every case, active samples have as a maximal protective effect the restoration of cell viability to the level seen in the absence of added TNF (i.e., the control labeled "cells alone").

The positive controls, r-hTBP-1 and TBP-IgG3, are both protective, showing a clear dose-dependence and ED50s of approximately 100 ng/ml for the r-hTBP-1 (Figs. 4-6) and about 1.5 ng/ml for TBP-IgG3 (Fig. 4) respectively.

The TBP-hCG constructs from 1x media (CHO or COS) or from the immunopurification show dose-dependent protection, with approximate ED50s ranging from 2-11 ng/ml (Figs. 4-6).

The results from the in vitro bioassay are reported in Table 5. The data indicate that the hybrid proteins inhibit TNF cytotoxicity, and that they are substantially more potent than the TBP monomer. The negative controls were devoid of protective activity.

In addition to the possibility that dimerization of TBP may increase potency, it is also possible that the activity of the hybrid proteins are not related to dimeric interaction with TBP, but rather to steric inhibition due to the partner of the hybrid interfering with soluble TBP/TNF binding to cell-surface TNF receptors.

### TABLES

| **Table 1: COS-7 transient expression (TBP ELISA)** | |
|---|---|
| **Hybrid Protein** | **Concentration (pg/ml)** |
| TBP1 | 66 |
| TBP-hCGα(20-161) | 5.1 |
| TBP-hCGβ(20-161) | 0.5 |
| TBP-hCG(20-161) | 2.7 |
| control | <0.25 |

| | |
|---|---|
| Constructs were expressed using pSVL (Pharmacia) | |

| **Table 2: COS-7 transient expression (TBP ELISA)** | |
|---|---|
| Hybrid protein | Concentration (ng/ml) |
| TBP1 | 131 |
| TBP-hCGα(20-190) | 81 |
| TBP-hCGβ(20-190) | 9 |
| TBP-hCG(20-190) | 62 |
| control | <1 |

| | |
|---|---|
| Constructs were expressed using a mouse metallothionein promoter-containing vector -pDα | |

| **Table 3: COS-7 transient expression (hCG heterodimer assay)** | |
|---|---|
| Hybrid Protein | Concentration (ng/ml) |
| TBP1 | <0.2 |
| TBP-hCGα(20-190) | <0.2 |
| TBP-hCGβ(20-190) | <0.2 |
| TBP-hCG(20-190) | 38 |
| control | <0.2 |

| | |
|---|---|
| Constructs were expressed using a mouse metallothionein promoter-containing vector - pDa | |

| **Table 4:Samples tested for anti-TNF activity** | | |
|---|---|---|
| *Construct* | *Cell source* | *Nature of sample* |
| r-hTBP-1 | CHO | purified |
| TBP-IgG3 | CHO | 1x conditioned media |
| TBP(20-161)-hCG | CHO | immunopurified (anti-TBP) |
| TBP(20-190)-hCG | CHO | 1x conditioned media |
| TBP(20-190)-hCG | COS | 1x conditioned media |

| **Table 5 :Preliminary Assessment of the hybrid proteins in TNF Cytotoxicity Assay** | | |
|---|---|---|
| *Construct* | *Fusion partner* | *Anti-TNF activity (ED50) in BT-20 bioassay⁻* |
| r-hTBP-1 | none | 100 ng/ml |
| TBP-IgG3 | IgG3 heavy chain constant region | 1.5 ng/ml |
| TBP(20-161)-hCG | hCGα and hCGβ (heterodimer) | 2 ng/ml |
| TBP(20-190)-hCG | hCGα and HCGβ (heterodimer) | 8-11 ng/ml |

| | | |
|---|---|---|
| **The quantitation of material for dosing and estimation of ED50 was made using the TBP ELISA. | | |

### REFERENCES

1. Smith, R.A. et al., J. Biol. Chem. 262:6951-6954, 1987.
2. Eck, M.J. et al., J. Biol. Chem. 264:17595-17605, 1989.
3. Jones, E.Y. et al, Nature 338:225-228, 1989.
4. Eck, M.J. et al., J. Biol. Chem. 267:2119-2122, 1992.
5. Pierce, J.G. et al., Annu. Rev. Biochem. 50:465-495, 1981.
6. Lapthorn, A.J. et al.; Nature 369:455-461, 1994.
7. Wu, H., et al., Structure 2:545-550, 1994.
8. Engelmann, H., et al., J. Biol. Chem. 265:14497-14504, 1990.
9. Adam, D. et al., J. Biol. Chem. 270:17482-17487, 1995.
10. Loetscher, H.R., et al., J. Biol. Chem. 266:18324-18329, 1991.
11. Banner, D.W., et al., Cell 73:431-445, 1993.
12. Pennica, D., et al., Biochemistry 32:3131-3138, 1993.
13. Engelmann, H. et al., J. Biol. Chem. 265:1531-1536, 1990.
14. Van Zee, K.J. et al., Proc. Natl. Acad. Sci. USA 89:4845-4849, 1992.
15. Aderka, D. et al., J. Exp. Med. 175:323-329, 1992.
16. Mohler, K.M., et al., J. Immunol. 151:1548-1561, 1993.
17. Bertini, R., et al., Eur. Cytokine Netw., 1993.
18. Piguet, P.F., et al., Immunology 77:510-514, 1992.
19. Williams, R.O., et al., Immunology 84:433-439, 1995.
20. Capon, D.J., et al., Nature 337: 525-531, 1989.
21. Ashkenazi, A., et al., Proc. Natl. Acad. Sci. 88:10535-10539, 1991.
22. Suitters, A.J., et al. J. Exp. Med. 179:849-856, 1994.
23. Nolan, O. et al., Biochim. Biophys. Acta 1040:1-11, 1990.
24. Rodrigues, M.L., et al., J. Immunol. 151:6954-6961, 1993.
25. Chang, H.-C., et al., Proc. Natl. Acad. Sci. USA 91:11408-11412, 1994.
26. Wu, Z., et al., J. Biol. Chem. 270:16039-16044, 1995.
27. Bazzoni, F. et al, Proc. Natl. Acad. Sci. USA 92:5376-5380, 1995.
28. Boldin, M.P., et al., J. Biol. Chem. 270:387-391, 1995.
29. Vu, T.-K.H., et al., Cell, 64:1057-1068, 1991.
30. Song, H.Y., et al.., J. Biol. Chem. 269:22492-22495, 1994.
31. Russell, D.A., et al., J. Infectious Diseases 171:1528-1538, 1995.
32. Rao C.V. et al., Am. J. Obstet. Gynecol., 146, 65-68, 1983.
33. Damewood M.D. et al., Fertil. Steril. 52, 398-400, 1989.
34. Chen, F., et al., Mol. Endocrinol. 6:914-919, 1992.
35. Bielinska, M., et al., J. Cell Biol. 11:330a, 1990.
36. Furuhashi, M., et al., Mol Endocrinol. 9:54-63, 1995.
37. Sugahara, T., et al., Pro. Natl. Acad. Sci. USA 92:2041-2045, 1995.
38. Johnson, G.A., et al., Biol. Reprod. 52:68-73, 1995.
39. Urlaub, G. and Chasin, L. Proc. Natl. Acad. Sci. USA 77:4216-4220, 1980.
40. Nophar, Y., et al., EMBO J. 9:3269-3278, 1990.
41. Fiddes, J.C. et al., Nature 281:351-356, 1979.
42. Fiddes, J.C. et al., Nature 286:684-687, 1980.
43. Elion, E.A., in Current Protocols in Molecular Biology, eds. Ausuble, FM. et al., John Wiley & Sons, 1993.
44. Campbell, R., Proc. Natl. Acad. Sci. USA 88:760-764, 1991.
45. Cole E.S. et al., Biotechnology, 11, 1014-1024, 1993.
46. Gluzman, Y., Cell 23:175-182, 1981.
47. Chu, G. et al., Nucl. Acid Res. 15:1311-1326, 1987.
48. Yen, J. et al., J. Immunotherapy 10:174-181, 1991.

## Claims

1. A hybrid protein comprising two coexpressed amino acid sequences forming a dimer, each comprising:
(a) at least one amino acid sequence selected from the group consisting of antibody light chains; antibody heavy chains; and antibody Fab domains; and
(b) a subunit of a heterodimeric proteinaceous hormone, or fragments thereof which retain the ability of the subunit to form a heterodimer with other subunits thereof;
wherein sequences (a) and (b) are bonded directly or through a peptide linker, and wherein in each couple the two subunits (b) are different and capable of aggregating to form a dimer complex.

2. The hybrid protein in accordance with claim 1, wherein said sequence (b) is selected from the group consisting of subunits of hCG, FSH, LH, TSH or inhibin, and fragments thereof.

3. The hybrid protein in accordance with claim 2, wherein said sequence (b) is modified to restrict its biological activity.

4. The hybrid protein in accordance with claim 2 or 3, wherein said sequence (b) is hCG and hCG has been mutagenized to eliminate its biological activity.

5. The hybrid protein in accordance with any one of claims 1 to 4, wherein sequence (a) is linked to the amino terminus, to the carboxy terminus, or to both termini of sequence (b).

6. The hybrid protein in accordance with any one of claims 1 to 5, wherein said hybrid protein is a monofunctional, bifunctional, or multifunctional molecule.

7. The hybrid protein in accordance with any one of claims 1 to 6, wherein said peptide linker has an enzyme cleavage site.

8. The hybrid protein in accordance with claim 7, wherein said enzyme cleavage site is a thrombin cleavage site.

9. The hybrid protein in accordance with claim 7, wherein said enzyme cleavage site is recognized and cleaved by an enzyme which is found in the ovary.

10. The hybrid protein in accordance with any one of claims 1 to 6, wherein said peptide linker serves as a flexible hinge.

11. The hybrid protein in accordance with any one of claims 1 to 10, wherein one or more covalent bonds between the two subunits (b) are added.

12. The hybrid protein in accordance with any one of claims 1 to 11, which is PEGylated.

13. A DNA molecule encoding one of the said two amino acid sequences forming a hybrid protein in accordance with any one of claims 1 to 12.

14. An expression vector containing one or two DNA molecules encoding amino acid sequences forming a hybrid protein in accordance with any one of claims 1 to 12 or a DNA molecule according to claim 13.

15. A host cell containing one or two different expression vector(s) in accordance with claim 14 and capable of expressing said hybrid protein.

16. The host cell in accordance with claim 15 which is a Chinese hamster ovary (CHO) cell.

17. A method for producing the hybrid protein of any one of claims 1 to 12 comprising culturing a host cell in accordance with claim 15 or 16.

18. The method of claim 17, further comprising recovering the hybrid protein.

19. The method of claim 17 or 18, further comprising formulating the hybrid protein in a pharmaceutical composition in combination with a pharmaceutically acceptable carrier and/or excipient.

20. A pharmaceutical composition comprising a hybrid protein in accordance with any one of claims 1 to 12 and a pharmaceutically acceptable carrier and/or excipient.

21. The hybrid protein of any one of claims 1 to 12 for use as a medicament.

## Patentansprüche

1. Hybridprotein, das zwei co-exprimierte, ein Dimer bildende Aminosäuresequenzen umfasst, die jeweils umfassen:
(a) mindestens eine Aminosäuresequenz, die ausgewählt ist aus der Gruppe bestehend aus leichten Ketten von Antikörpern, schweren Ketten von Antikörpern und Fab-Domänen von Antikörpern; und
(b) eine Untereinheit eines heterodimeren Proteinhormons oder Fragmenten davon, die die Fähigkeit der Untereinheit behalten, ein Heterodimer mit anderen Untereinheiten davon zu bilden;
wobei die Sequenzen (a) und (b) direkt über einen Peptidlinker verbunden sind, und wobei in jedem Paar die beiden Untereinheiten (b) verschieden sind und fähig sind, unter Bildung eines dimeren Komplexes zu aggregieren.

2. Hybridprotein nach Anspruch 1, wobei die Sequenz (b) ausgewählt ist aus der Gruppe bestehend aus Untereinheiten von hCG, FSH, LH, TSH oder Inhibin und Fragmenten davon.

3. Hybridprotein nach Anspruch 2, wobei die Sequenz (b) modifiziert ist, um ihre biologische Aktivität einzuschränken.

4. Hybridprotein nach Anspruch 2 oder 3, wobei die Sequenz (b) hCG ist und hCG mutagenisiert wurde, um dessen biologische Aktivität auszuschalten.

5. Hybridprotein nach einem der Ansprüche 1 bis 4, wobei die Sequenz (a) mit dem Amino-Terminus, dem Carboxy-Terminus oder mit beiden Termini der Sequenz (b) verbunden ist.

6. Hybridprotein nach einem der Ansprüche 1 bis 5, wobei das Hybridprotein ein monofunktionales, bifunktionales oder multifunktionales Molekül ist.

7. Hybridprotein nach einem der Ansprüche 1 bis 6, wobei der Peptidlinker eine Enzymspaltstelle aufweist.

8. Hybridprotein nach Anspruch 7, wobei die Enzymspaltstelle eine Thrombinspaltstelle ist.

9. Hybridprotein nach Anspruch 7, wobei die Enzymspaltstelle von einem Enzym, erkannt und gespalten wird, das im Eierstock vorkommt.

10. Hybridprotein nach einem der Ansprüche 1 bis 6, wobei der Peptidlinker als flexibles Gelenk dient.

11. Hybridprotein nach einem der Ansprüche 1 bis 10, wobei eine oder mehrere kovalente Bindungen zwischen den beiden Untereinheiten (b) hinzugefügt sind.

12. Hybridprotein nach einem der Ansprüche 1 bis 11, das PEGyliert ist.

13. DNA-Molekül, das eine der beiden Aminosäuresequenzen codiert, die ein Hybridprotein nach einem der Ansprüche 1 bis 12 bilden.

14. Expressionsvektor, der ein oder zwei DNA-Moleküle enthält, die Aminosäuresequenzen codieren, die ein Hybridprotein nach einem der Ansprüche 1 bis 12 bilden, oder ein DNA-Molekül nach Anspruch 13.

15. Wirtszelle, die einen oder zwei verschiedene Expessionsvektor(en) nach Anspruch14 enthält und zur Expression des Hybridproteins fähig ist.

16. Wirtszelle nach Anspruch 15, die eine Ovarialzelle des chinesischen Hamsters (CHO-Zelle) ist.

17. Verfahren zur Herstellung des Hybridproteins nach einem der Ansprüche 1 bis 12, umfassend das Züchten einer Wirtszelle nach Anspruch 15 oder 16.

18. Verfahren nach Anspruch 17, des Weiteren umfassend das Gewinnen des Hybridproteins.

19. Verfahren nach Anspruch 17 oder 18, des Weiteren umfassend das Formulieren des Hybridproteins in einem Arzneimittel in Kombination mit einem pharmazeutisch verträglichen Träger und/oder Excipienten.

20. Arzneimittel, umfassend ein Hybridprotein nach einem der Ansprüche 1 bis 12 und einen pharmazeutischen Träger und/oder Excipienten.

21. Hybridprotein nach einem der Ansprüche 1 bis 12 zur Verwendung als ein Medikament.

## Revendications

1. Protéine hybride comprenant deux séquences d'acides aminés co-exprimées formant un dimère, dont chacune comprend :
a) au moins une séquence d'acides aminés choisie dans l'ensemble formé par les chaînes légères d'anticorps, les chaînes lourdes d'anti-corps et les fragments Fab d'anticorps ;
b) et une sous-unité d'une hormone protéique hétérodimère, ou des fragments d'une telle sous-unité qui conservent la capacité de la sous-unité à former un hétérodimère avec d'autres sous-unités de celle-là ;
dans laquelle les séquences (a) et (b) sont reliées directement ou par l'intermédiaire d'un raccord peptidique,
et dans laquelle les deux sous-unités (b) sont différentes d'un couple à l'autre et sont capables de s'agréger pour former un complexe dimère.

2. Protéine hybride conforme à là revendication 1, dans laquelle ladite séquence (b) est choisie dans l'ensemble formé par les sous-unités des hormones hCG, FSH, LH, TSH et de l'inhibine, ainsi que les fragments de ces sous-unités.

3. Protéine hybride conforme à la revendication 2, dans laquelle ladite séquence (b) est modifiée de manière à ce que son activité biologique soit limitée.

4. Protéine hybride conforme à la revendication 2 ou 3, dans laquelle ladite séquence (b) est une hormone hCG à laquelle on a fait subir une mutagenèse pour en annihiler l'activité biologique.

5. Protéine hybride conforme à l'une des revendications 1 à 4, dans laquelle la séquence (a) est attachée à l'extrémité amino-terminale, à l'extrémité carboxy-terminale, ou au deux extrémités, de la séquence (b).

6. Protéine hybride conforme à l'une des revendications 1 à 5, laquelle protéine hybride est une molécule monofonctionnelle, bifonctionnelle ou multifonctionnelle.

7. Protéine hybride conforme à l'une des revendications 1 à 6, dans laquelle ledit raccord peptidique comporte un site de clivage enzymatique.

8. Protéine hybride conforme à la revendication 7, dans laquelle ledit site de clivage enzymatique est un site de clivage par la thrombine.

9. Protéine hybride conforme à la revendication 7, dans laquelle ledit site de clivage enzymatique est un site qui est reconnu par une enzyme qui se trouve dans les ovaires et au niveau duquel celle-ci opère un clivage.

10. Protéine hybride conforme à l'une des revendications 1 à 6, dans laquelle ledit raccord peptidique sert d'articulation flexible.

11. Protéine hybride conforme à l'une des revendications 1 à 10, dans laquelle il y a en plus une ou plusieurs liaisons covalentes établies entre les deux sous-unités (b).

12. Protéine hybride conforme à l'une des revendications 1 à 11, qui est pégylée.

13. Molécule d'ADN codant l'une des deux séquences d'acides aminés susdites qui forment une protéine hybride conforme à l'une des revendications 1 à 12.

14. Vecteur d'expression comportant une ou deux molécule(s) d'ADN codant des séquences d'acides aminés qui forment une protéine hybride conforme à l'une des revendications 1 à 12, ou une molécule d'ADN conforme à la revendication 13.

15. Cellule hôte contenant un vecteur d'expression ou deux vecteurs d'expression différents, conforme(s) à la revendication 14 et capable(s) d'exprimer ladite protéine hybride.

16. Cellule hôte conforme à la revendication 15, qui est une cellule CHO (cellule d'ovaire de hamster chinois).

17. Procédé de production d'une protéine hybride conforme à l'une des revendications 1 à 12, qui comporte le fait de cultiver une cellule hôte conforme à la revendication 15 ou 16.

18. Procédé conforme à la revendication 17, qui comporte en outre le fait de récupérer la protéine hybride.

19. Procédé conforme à la revendication 17 ou 18, qui comporte en outre le fait de mettre la protéine hybride en formulation dans une composition pharmaceutique, en association avec un véhicule et/ou un excipient pharmacologiquement admissible(s).

20. Composition pharmaceutique comprenant une protéine hybride conforme à l'une des revendications 1 à 12, et un véhicule et/ou un excipient pharmacologiquement admissible(s).

21. Protéine hybride conforme à l'une des revendications 1 à 12, pour utilisation en tant que médicament.
